(19) Europäisches Patentamt
European Patent Office
Office européen des brevets

(11) EP 4 056 575 A1

(12) EUROPEAN PATENT APPLICATION
published in accordance with Art. 153(4) EPC

(43) Date of publication:
**14.09.2022 Bulletin 2022/37**

(21) Application number: **20885710.2**

(22) Date of filing: **04.11.2020**

(51) International Patent Classification (IPC):
*C07D 498/04* (2006.01) *C07D 498/14* (2006.01)
*C07D 519/00* (2006.01) *A61P 35/00* (2006.01)
*A61K 31/4188* (2006.01) *A61K 31/4196* (2006.01)
*A61K 31/4162* (2006.01)

(52) Cooperative Patent Classification (CPC):
**A61K 31/4162; A61K 31/4188; A61K 31/4196; A61P 35/00; C07D 498/04; C07D 498/14; C07D 519/00**

(86) International application number:
**PCT/CN2020/126359**

(87) International publication number:
**WO 2021/088839 (14.05.2021 Gazette 2021/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2019 CN 201911066776**
**28.10.2020 CN 202011167875**

(71) Applicant: **Betta Pharmaceuticals Co., Ltd**
**Yuhang**
**Hangzhou**
**Zhejiang 311100 (CN)**

(72) Inventors:
- **ZU, Houxian**
  **Beijing 100176 (CN)**
- **SONG, Xizhen**
  **Beijing 100176 (CN)**
- **CHEN, Kai**
  **Beijing 100176 (CN)**
- **LIU, Xiangyong**
  **Beijing 100176 (CN)**
- **CHEN, Jie**
  **Beijing 100176 (CN)**
- **BIAN, Yajing**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **IPAZ**
**Bâtiment Platon**
**Parc Les Algorithmes**
**91190 Saint-Aubin (FR)**

# (54) IMIDAZOLIDINONE COMPOUND, PREPARATION METHOD THEREFOR AND USE THEREOF

(57) An imidazolidinone compound represented by formula (I), or a stereoisomer thereof, a geometric isomer thereof, or a tautomer thereof, or a pharmaceutically acceptable salt thereof, a pharmaceutical composition containing the compound, a synthesis method therefor and use thereof.

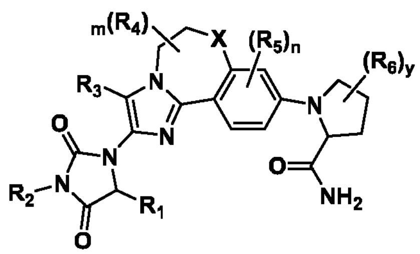

Formula (I)



Processed by Luminess, 75001 PARIS (FR)

## Description

## FIELD OF THE INVENTION

The present invention relates to imidazolidinone compounds, which play a role by participating in the regulation of multiple processes such as cell proliferation, apoptosis, migration, and angiogenesis. The present invention also relates to the pharmaceutical compositions comprising such compounds and the use of said compounds for the treatment of the disease mediated by PI3K.

## BACKGROUND OF THE INVENTION

PI3K pathway is important for regulating the intracellular activities such as growth, proliferation, survival, differentiation, metastasis and apoptosis. It is called PI3K/Akt/mTOR signaling pathway because PI3K, Akt and mTOR are key proteins in this pathway. In recent years, PI3K inhibitor has been a research hotspot of antitumor drug.

After activated by RTK or Ras, PI3K catalyzes phosphoinositide-3, 4-bisphosphate (PIP2) to phosphoinositide-3, 4, 5-triphosphate (PIP3). PIP3 binds to protein kinases such as Akt and 3-phosphoinositide (PIP)-dependent protein kinase (PDK), phosphorylates and activates Akt, and then translocates Akt from cytoplasm to nucleus. Activated Akt further phosphorylate downstream effector substrates to affect cell survival, cell cycle, growth and other cellular activities. (Ma K, Cheung SM, Marshall AJ etc., Cell Signal, 2008, 20:684-694). Therefore, activation of the PI3K/Akt/mTOR signaling pathway can inhibit cell apoptosis, enhance cell tolerance, promote cell survival, proliferation and participate in angiogenesis, promote tumor growth and metastasis.

Phosphatidylinositol 3-kinase (PI3K) belongs to lipid kinase family, whichis divided into class I, class II and class III based on activation mechanism and structural features (Vanhaesebroeck B, Waterfield MD; Exp Cell Res, 1999, 253:239-254). ClassPI3Ks are the most well-studied at present. Class I PI3Ks are further divided into IA and IB isoforms, which receive signals transduction from receptor tyrosine kinase (RTKs) and G protein-coupled receptors (GPCRs) respectively (Wu P, Liu T, Hu Y; Curr Med Chem, 2009, 16:916-930). Class IA PI3Ks comprise three isoforms designated PI3Kα, PI3Kβ and PI3Kδ, Class IB PI3Ks only comprise one isoform (PI3Kγ). Class II PI3Ks are divided into three catalytic isoforms designated PI3KC2α, PI3KC2β and PI3KC2γ based on different C-terminal structure. However, little is known about their substrates in vivo, and the understanding of their mechanisms of action and specific function is relatively limited. (Falasca M, T. Muffucci; Biochem Soc Trans, 2007, 35:211-214). Class III PI3Ks only comprise one member, Vps34 (Vacuolar Protein Sorting 34), which acts as a calibrator at the protein level in regulating downstream mTOR signaling cascades (Schu P, Takegawa. K, Fry. M etc., Science, 1993, 260:88-91).

Among the four class I PI3Ks, PI3Kα and PI3Kβ are expressed in various organs, but PI3Kδ and PI3Kγ are mainly distributed in the bone marrow cells (Kong D, Yamori T; cancer Sci, 2008, 99:1734-1740). PI3Kα is the most closely related to the development of tumor, the gene encoding the catalytic subunit p110α of PI3Kα is *PIK3CA,* and its mutation are frequently observed in various malignancies including breast, colon, endometrial, stomach, ovarian and lung cancer ect (Steelman. LS, Chappell. WH, Abrams. SLet al., Aging, 2011, 3:192-222). The aberrant activation of PI3Kα upregulates and activates the PI3K signaling pathway, which can promote cell hyperproliferation, overgrowth and metastasis and then form the tumor. Although the other three isoforms PI3Kβ, PI3Kδ and PI3Kγ play roles in thrombosis, immune function, allergic and inflammatory responses respectively, they are also important in tumor development by influencing the catalytic activity, physicochemical properties, interactions and recognition.

The most studied PI3K inhibitors in the early stage are wortmannin and LY294002, which play an important role in the study on the physiological functions and the mechanisms of action of the signaling pathway of PI3K. They are called the first generation of PI3K inhibitors which provide an important foundation for subsequent research. Based on the wortmannin and LY294002, the second generation of PI3K inhibitors such as morpholine aryls, imidazopyridines and imidazoquinolines, with new structures, higher activities and better pharmacokinetic properties were discoverd, which brings new hope to tumor therapy.There have been dozens of PI3K inhibitors under clinical investigation, mainly include pan-PI3K inhibitors, PI3K/mTOR dual inhibitors, and PI3K isoform-specific inhibitors.

Alpelisib (BYL719) is the first PI3Kα selective inhibitor developed by Norvartis, with an inhibitory activity against p110α at 5 nM.The preclinical data shows that BYL719 inhibits phosphorylation of Akt, blocks the PI3K signaling pathway and inhibits proliferation of breast cancer cells carring *PIK3CA* mutations. (Dejan Juric etc., cancer Res, 2012, 72:1). The drug has been approved by the U.S. Food and Drug Administration (FDA) on May 24, 2019, for the treatment of postmenopausal female and male with HR+/HER2-advanced or metastatic breast cancer with *PIK3CA* mutations and disease progression during or after endocrine therapy regimens. At the same time, the previous research indicates that the PI3Ka-specific small molecule inhibitor shows promising therapeutic effects for head and neck cancer, ovarian cancer, triple negative breast cancer, HER2+ breast cancer and *PIK3CA-related* overgrowth spectrumother (PROS). Indication expansion of the drug, will create huge economic and social benefits.

To achieve a better tumor treatment effect, meet the market better, and provide an alternative for clinical use,

we developed a new generation of PI3K inhibitor with higher activity, better pharmacokinetic properties and lower toxicity.

## SUMMARY OF THE INVENTION

The present invention provides a series of imidazolidinone compounds as PI3K inhibitor.

The present invention provides a compound of Formula I, or a stereoisomer, geometric isomer, tautomer, pharmaceutically acceptable salt, solvate, chelate, non-covalent complex, or prodrug thereof,

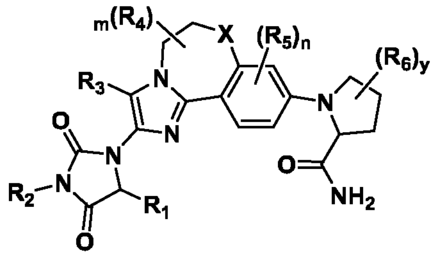

Formular (I)

wherein,

X is selected from O and S;
$R_1$ is selected from H, CN, $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl, $C_{5-8}$ heteroaryl, $OR_a$ and $-NR_aR_b$; wherein $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, $OR_a$, oxo, $C_{1-6}$ alkyl, $C_{2-6}$ alkenyl, $C_{2-6}$ alkynyl, $C_{3-6}$ cycloalkyl and $C_{3-6}$ heterocyclyl;
$R_2$ is selected from H, $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl, $C_{5-8}$ heteroaryl; wherein $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, -OH, $-NO_2$, $C_{1-6}$ alkyl, $C_{1-6}$ haloalkyl, $C_{2-6}$ alkenyl, $C_{2-6}$ haloalkenyl, $C_{2-6}$ alkynyl, $C_{2-6}$ haloalkynyl, $C_{3-6}$ cycloalkyl, $C_{3-6}$ halocycloalkyl, $C_{3-6}$ heterocyclyl, $C_{3-6}$ haloheterocyclyl, $C_{6-8}$ aryl, $C_{6-8}$ haloaryl, $C_{5-8}$ heteroaryl, $C_{5-8}$ haloheteroaryl, oxo, $-OR_a$, $-NR_aR_b$, $-C(O)R_a$, $-C(O)OR_a$, $-C(O)NR_aR_b$, $- S(O)R_a$ and $-S(O)_2R_a$;
$R_3$ is selected from H, halogen, CN, $C_{1-6}$ alkyl, $C_{2-6}$ alkenyl, $C_{2-6}$ alkynyl, $-OR_a$ and $- NR_aR_b$; wherein $C_{1-6}$ alkyl, $C_{2-6}$ alkenyl, $C_{2-6}$ alkynyl are each optionally substituted with one or more substituents selected from halogen, CN, $-OR_a$, $-NR_aR_b$, $-C(O)R_a$, $-C(O)OR_a$, $- C(O)NR_aR_b$, $-S(O)R_a$ and $-S(O)_2R_a$;
$R_4$ is selected from H, halogen, CN, $C_{1-6}$ alkyl, $C_{2-6}$ alkenyl, $C_{2-6}$ alkynyl, oxo, $C_{1-6}$ haloalkyl, $C_{2-6}$ haloalkenyl, $C_{2-6}$ haloalkynyl, $C_{1-6}$ alkoxy, $C_{1-6}$ haloalkoxy, $-OR_a$, $-NR_aR_b$, $- C(O)R_a$, $-C(O)OR_a$, $-C(O)NR_aR_b$, $-S(O)R_a$ and $-S(O)_2R_a$;
$R_5$ is selected from H, halogen, CN, $C_{1-6}$ alkyl, $C_{2-6}$ alkenyl, $C_{2-6}$ alkynyl, $C_{1-6}$ haloalkyl, $C_{2-6}$ haloalkenyl, $C_{2-6}$ haloalkynyl, $C_{1-6}$ alkoxy, $C_{1-6}$ haloalkoxy, $-OR_a$, $-NR_aR_b$, $- S(O)R_a$ and $-S(O)_2R_a$;
$R_6$ is selected from H, halogen, CN, oxo, $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl, $C_{5-8}$ heteroaryl, $-OR_a$, $-NR_aR_b$, $-C(O)R_a$, $-C(O)O R_a$, $-C(O)NR_aR_b$, $-S(O)R_a$ and $-S(O)_2R_a$; wherein $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, oxo, $-NO_2$, $C_{1-6}$ alkyl, $C_{2-6}$ alkenyl, $C_{2-6}$ alkynyl, $-OR_a$, $-NR_aR_b$, $-C(O)R_a$, $-C(O)OR_a$, $- C(O)NR_aR_b$, $-S(O)R_a$ and $-S(O)_2R_a$; or,
Two $R_6$ taken together with the C atoms which they are attached form a $C_{3-6}$ cycloalkyl or $C_{3-6}$ heterocyclyl;
$R_a$ and $R_b$ are independently selected from H, $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl, $C_{5-8}$ heteroaryl; wherein $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl, $C_{5-8}$ heteroaryl are each optionally substituted with halogen, CN, -OH, $-NH_2$, $C_{1-6}$alkyl, $C_{1-6}$ alkoxy, $C_{1-6}$ haloalkyl, $C_{1-6}$ haloalkoxy;
m is selected from 0, 1, 2, 3 and 4;
n is selected from 0, 1, 2 and 3;
y is selected from 0, 1, 2, 3, 4, 5 and 6.

The present invention provides some preferred technical solution about the compound of Formula (I), or a stereoisomer, geometric isomer or tautomer thereof:

In some embodiments, $R_1$ is $C_{1-6}$ alkyl or $C_{3-6}$ cycloalkyl, wherein $C_{1-6}$ alkyl and $C_{3-6}$ cycloalkyl are each independently and optionally substituted with halogen.

In some embodiments, $R_2$ is selected from H, $C_{1-6}$ alkyl, $C_{3-6}$ cycloalkyl, $C_{3-6}$ heterocyclyl, $C_{6-8}$ aryl and $C_{5-8}$

heteroaryl; wherein $C_{1-6}$ alkyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, -CN, -OH, $-NR_aR_b$, $C_{1-6}$ alkyl and $C_{1-6}$ haloalkyl; wherein $R_a$ and $R_b$ are independently selected from H and $C_{1-6}$ alkyl.

In some embodiments, $R_3$ is selected from H, halogen, CN, $C_{1-6}$ alkyl, $C_{2-6}$ alkenyl, $C_{2-6}$ alkynyl, $C_{1-6}$ haloalkyl, $C_{2-6}$ haloalkenyl and $C_{2-6}$ haloalkynyl.

In some embodiments, $R_6$ is selected from H, halogen, CN, $-NH_2$, oxo, -OH, $C_{1-6}$ alkyl, $C_{2-6}$ alkenyl, $C_{2-6}$ alkynyl, $C_{1-6}$ alkoxy, $C_{1-6}$ haloalkyl, $C_{2-6}$ haloalkenyl, $C_{2-6}$ haloalkynyl, $C_{3-6}$ cycloalkyl, $C_{3-6}$ heterocyclyl, $C_{6-8}$ aryl and $C_{5-8}$ heteroaryl.

In some embodiments, the compound of Formula (I) is further of Formula (II):

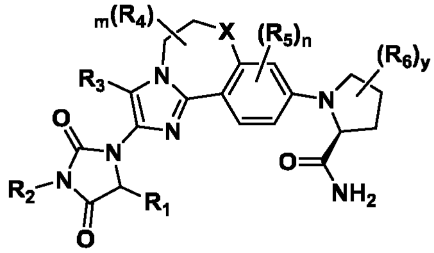

Formula (II).

In some embodiments, the compound of Formula (I) is further of Formula (III-1):

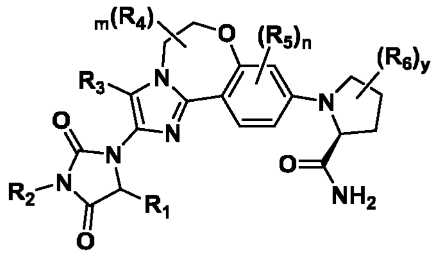

Formula (III-1)

wherein,

$R_1$ is $C_{1-6}$ alkyl or $C_{1-6}$ haloalkyl.

In some embodiments, the compound of Formula (I) is further of Formula (III-2):

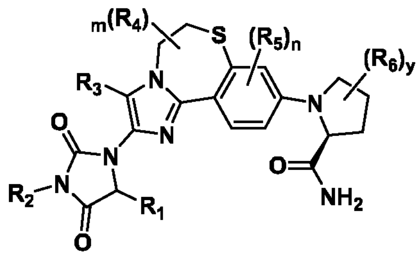

Formula (III-2).

In some embodiments, $R_3$ is selected from H, halogen, $C_{1-6}$ alkyl and $C_{2-6}$ alkenyl.

In some embodiments, $R_4$ and $R_5$ are both H.

In some embodiments, the compound of Formula (I) is further of Formula (IV):

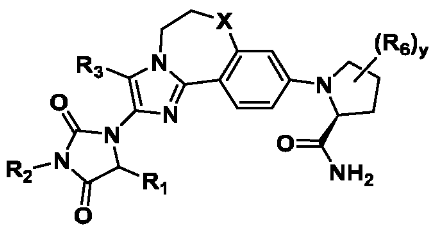

Formula (IV)

Wherein:

$R_1$ is $C_{1-6}$ alkyl or $C_{1-6}$ haloalkyl;
$R_2$ is selected from H, $C_{1-6}$ alkyl, $C_{3-6}$ cycloalkyl, $C_{3-6}$ heterocyclyl, $C_{6-8}$ aryl or $C_{5-8}$ heteroaryl; wherein $C_{1-6}$ alkyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, -CN, -OH, $-NR_aR_b$, $C_{1-6}$ alkyl, $C_{1-6}$ haloalkyl, $C_{1-6}$ alkoxy and $C_{1-6}$ haloalkoxy;
$R_3$ is selected from H, halogen, $C_{1-6}$ alkyl and $C_{2-6}$ alkenyl;
$R_6$ is selected from H, halogen, $-NH_2$, oxo, -OH, $C_{1-6}$ alkyl, $C_{1-6}$ alkoxy, $C_{1-6}$ haloalkyl, $C_{1-6}$ haloalkoxy, $C_{3-6}$ cycloalkyl and $C_{6-8}$ aryl;
$R_a$ and $R_b$ are independently selected from H and $C_{1-6}$ alkyl;
Y is selected from 0, 1, 2, 3, 4, 5 and 6.

In some embodiments, wherein X is O.

In some embodiments, the compound of Formula (I) is further of Formula (V):

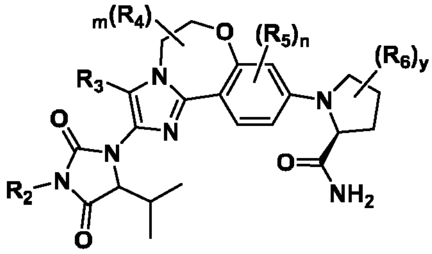

Formula (V).

In some embodiments, $R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $C_{1-6}$ alkyl, $C_{3-6}$ cycloalkyl, $C_{3-6}$ heterocyclyl, $C_6$ aryl and $C_{5-6}$ heteroaryl are each optionally substituted with one or more substituents selected from halogen, -CN, -OH, $-N-(CH_3)_2$, $C_{1-6}$ alkyl and $C_{1-6}$ haloalkyl.

In some embodiments, $R_6$ is selected from H, halogen, $-NH_2$, oxo, -OH, $C_{1-6}$ alkyl, $C_{1-6}$ alkoxy, $C_{3-6}$ cycloalkyl and $C_6$ aryl.

In some embodiments, the compound of Formula (I) is further of Formula (VI):

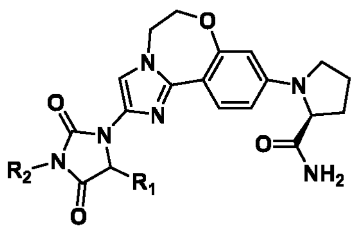

Formula (VI)

wherein,

$R_1$ is selected from $C_{1-6}$ alkyl and $C_{1-6}$ haloalkyl;
$R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $C_{1-6}$ alkyl, $C_{3-6}$ cycloalkyl, $C_{3-6}$ heterocyclyl, $C_6$ aryl and $C_{5-6}$ heteroaryl are optionally substituted with one or more substituents selected from halogen, -CN, -OH, $-N-(CH_3)_2$, $C_{1-6}$ alkyl and $C_{1-6}$ haloalkyl.

In some embodiments, $R_1$ is $C_{1-4}$ alkyl; $R_2$ is selected from H, $C_{1-6}$ alkyl, $C_{3-6}$ cycloalkyl, $C_{3-6}$ heterocyclyl, $C_6$ aryl and $C_5$-$C_6$ heteroaryl; wherein $R_1$ and $R_2$ are independently and optionally substituted with halogen.
In some embodiments, $R_2$ is selected from $C_{1-6}$ alkyl, $C_{1-6}$ haloalkyl, $C_{3-6}$ cycloalkyl, $C_{3-6}$ halocycloalkyl, phenyl and halogen substituted phenyl.
In some embodiments, $R_1$ is selected from $-CH(CH_3)_2$, $-C(CH_3)_3$, $-CF_3$ and $-CHF_2$.
In some embodiments, the compound of Formula (I) is further of Formula (VII):

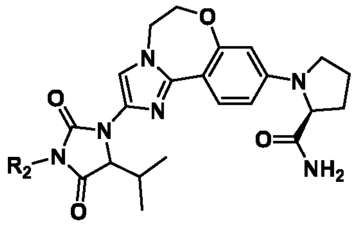

Formula (VII)

wherein, $R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $R_2$ is optionally substituted with halogen.
In some embodiments, $R_1$ is $C_{1-4}$ alkyl; $R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $R_1$ and $R_2$ are independently and optionally substituted with F.
In some embodiments, $R_2$ is selected from $-CH_3$, $-CH_2CH_3$, $-CH(CH_3)_2$, $-CH_2CF_3$, cyclopropyl, cyclobutyl, phenyl and pyridyl; wherein the $-CH_3$, $-CH_2CH_3$, $-CH(CH_3)_2$, - $CH_2CF_3$, cyclopropyl, cyclobutyl, phenyl or pyridyl are optionally substituted with halogen.
In some embodiments, halogen is F.
In some embodiments, $R_2$ is selected from $-CH_3$, $-CH(CH_3)_2$, cyclopropyl, phenyl and halogen substituted phenyl.
In some embodiments, $R_1$ is $-CH(CH_3)_2$.
In some embodiments, $R_1$ is-$CHF_2$.
In some embodiments, $R_2$ is selected from $-CH_3$, $-CH(CH_3)_2$, cyclopropyl, phenyl or F substituted phenyl, pyridyl and F substituted pyridyl .
In some embodiments, $R_2$ is selected from $-CH_3$, $-CH(CH_3)_2$, cyclopropyl, phenyland F substituted phenyl.
In some embodiments, $R_2$ is $-CH(CH_3)_2$ or cyclopropyl.
In some embodiments, the compound of Formula (I) is further of Formula (VIII):

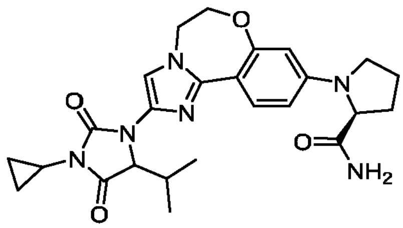

Formula (VIII)

The present invention provides the most preferred technical solutions about a compound of Formula (I), or stereoisomer, geometric isomer or tautomer thereof, wherein the compounds are:

1) (2S)-1-(2-(5-isopropyl-3-methyl-2,4-dioxoimidazolidin-1-yl)-5,6-dihydrobenzo[f]imidazo[1,2-d] [1,4] oxazepin-9-yl)pyrrolidine-2-carboxamide;
2) (2S)-1-(2-(3-cyclopropyl-5-isopropyl-2,4-dioxoimidazolidin-1-yl)-5,6-dihydrobenzo[f]imidazo[1,2-d] [1,4] oxazepin-9-yl)pyrrolidine-2-carboxamide;
3) (2S)-1-(2-(5-isopropyl-2,4-dioxo-3-(2,2,2-trifluoroethyl)imidazolidine-1-yl)-5,6-dihydrobenzo[f]imidazo[1,2-d][1,4]oxazepin-9-yl)pyrrolidine-2-carboxamide;
4) (2S)-1-(2-(3-(4-fluorophenyl)-5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
5) (2S)-1-(2-(5-cyclopropyl -3-methyl-2,4-dioxoimidazolidin-1-yl)-5,6-dihydrobenzo[f]imidazo[1,2-d] [1,4] oxazepin-9-yl)pyrrolidine-2-carboxamide;
6) (2S)-1-(2-(3-cyclobutyl-5-isopropyl-2,4-dioxoimidazolidin-1-yl)-5,6-dihydrobenzo[f]imidazo[1,2-d] [1,4] oxazepin-9-yl)pyrrolidine-2-carboxamide;
7) (2S)-1-(2-(3-ethyl-5-isopropyl-2,4-dioxoimidazolidin-1-yl)-5,6-dihydrobenzo[f]imidazo[1,2-d] [1,4] oxazepin-9-yl)pyrrolidine-2-carboxamide;
8) (2S)-1-(2 -(5-difluoromethyl-3-methyl-2,4-dioxoimidazolidin-1-yl)-5,6-dihydrobenzo[f]imidazo[1,2-d] [1,4] oxazepin-9-yl)pyrrolidine-2-carboxamide;
9) (2S)-1-(2-(5-isopropyl-2,4-dioxo-3-phenylimidazolidine-1-yl)-5,6-dihydrobenzo[f]imidazo[1,2-d] [1,4] oxazepin-9-yl)pyrrolidine-2-carboxamide;
10) (S)-1-(2-((S)-5-isopropyl-2,4-dioxo-3-phenylimidazolidine-1-yl)-5,6-dihydrobenzo[f]imidazo[1,2-d] [1,4]oxazepin-9-yl)pyrrolidine-2-carboxamide;
11) (S)-1-(2-((R)-5-isopropyl-2,4-dioxo-3-phenylimidazolidine-1-yl)-5,6-dihydrobenzo[f]imidazo[1,2-d] [1,4]oxazepin-9-yl)pyrrolidine-2-carboxamide;
12) (2S)-1-(2-(5-isopropyl-2,4-dioxo-3-(4-(trifluoromethyl)phenyl)imidazolidine-1-yl)-5,6-dihydrobenzo[f]imidazo[1,2 -d] [1,4] oxazepin-9-yl)pyrrolidine-2-carboxamide;
13) (2S)-1-(2-(5-isopropyl-2,4-dioxo-3-propylimidazolidine-1-yl)-5,6-dihydrobenzo[f]imidazo[1,2-d] [1,4] oxazepin-9-yl)pyrrolidine-2-carboxamide;
14) (2S)-1-(2-(3-cyclopropyl -5-isopropyl)-2,4-dioxoimidazolidin-1-yl)-5,6-dihydrobenzo[f]imidazo[1,2-d] [1,4] oxazepin-9-yl)-2-methylpyrrolidine-2-carboxamide;
15) (2S)-1-(2-(3-cyclopropyl -5-isopropy)-2,4-dioxoimidazolidin-1-yl)-5,6-dihydrobenzo[f]imidazo[1,2-d] [1,4] thiazepin-9-yl)-4,4-difluoropyrrolidine-2-carboxamide;
16) (2S)-1-(2-(5-isopropyl-3-(1-methyl-1H-1,2,4-triazol-5-yl)-2,4-dioxoimidazolidin-1-yl)-5,6 -dihydrobenzo[f]imidazo[1,2-d] [1,4]oxazepin-9-yl)pyrrolidine-2-carboxamide;
17) (2S,4S)-4-amino-1-(2-(3-cyclopropyl -5-isopropyl-2,4-dioxoimidazolidin-1-yl)-5,6-dihydrobenzo[f] imidazo [1,2-d] [1,4]oxazepin-9-yl)pyrrolidine-2-carboxamide;
18) (2S)-1-(2-(3-(2-fluorophenyl)-5-isopropyl-2,4-dioxoimidazolidin-1-yl)-5,6-dihydrobenzo[f]imidazo[1,2-d] [1,4]oxazepin-9-yl)pyrrolidine-2-carboxamide;
19) (2S)-1-(2-(3-(4-chlorophenyl)-5-isopropyl-2,4-dioxoimidazolidin-1-yl)-5,6-dihydrobenzo[f]imidazo[1,2-d] [1,4] oxazepin-9-yl)pyrrolidine-2-carboxamide;
20) (2S)-4-(tertbutoxy)-1-(2-(3-cyclopropyl -5-isopropyl-2,4-dioxoimidazolidin-1-yl)-5,6-dihydrobenzo[f] imidazo [1,2] -d] [1,4]oxazepin-9-yl)pyrrolidine-2-carboxamide;
21) (2S)-1-(2-(3-cyclopropyl -5-isopropyl)-2,4-dioxoimidazolidin-1-yl)-5,6-dihydrobenzo[f]imidazo[1,2-d] [1,4]oxazepin-9-yl)-4,4-dimethylpyrrolidine-2-carboxamide;
22) (2S)-1-(2-(3-(2,2-difluoroethyl)-5-isopropyl-2,4-dioxoimidazolidin-1-yl)-5,6-dihydrobenzo[f]imidazo[1,2-d ] [1,4] oxazepin-9-yl)pyrrolidine-2-carboxamide;
23) (2S)-1-(2-(3,5-diisopropyl-2,4-dioxoimidazolidin-1-yl)-5,6-dihydrobenzo[f]imidazo[1,2-d] [1,4] oxazepin- 9-yl)pyrrolidine-2-carboxamide;
24) (2S)-1-(2-(3-cyclopropyl -5-isopropyl)-2,4-dioxoimidazolidin-1-yl)-5,6-dihydrobenzo[f]imidazo[1,2-d] [1,4]oxazepin-9-yl)-4-phenylpyrrolidine-2-carboxamide;
25) (2S)-1-(2-(3-(3-chloro5-cyanophenyl)-5-isopropyl-2,4-dioxoimidazolidin-1-yl)-5,6-dihydrobenzo[f]imidazo[1,2 -d] [1,4] thiazepin-9-yl)pyrrolidine-2-carboxamide;
26) (2S)-1-(2-(3,5-diisopropyl-2,4-dioxoimidazolidin-1-yl)-5,6-dihydrobenzo[f]imidazo[1,2-d] [1,4]thiazepin- 9-yl)pyrrolidine-2-carboxamide;
27) (2S)-1-(2- (3-azetidin-3-yl)-5-isopropyl-2,4-dioxoimidazolidin-1-yl)-5,6-dihydrobenzo[f]imidazo[1,2-d] [1,4] oxazepin-9-yl)pyrrolidine-2-carboxamide;
28) (2S)-1-(2-(3-methyl-2,4-dioxoimidazolidin-1-yl)-5,6-dihydrobenzo[f]imidazo[1,2-d] [1,4] oxazepin-9-yl)pyrrolidine-2-carboxamide;
29) (2S)-1-(2- (5-tertbutyl-3-methyl-2,4-dioxoimidazolidin-1-yl)-5,6-dihydrobenzo[f]imidazo[1,2-d] [1,4] oxazepin-9-yl)pyrrolidine-2-carboxamide;
30) (S)-1-(2-((R)-3-(4-fluorophenyl)-5-isopropyl-2,4-dioxoimidazolidin-1-yl)-5,6-dihydrobenzo[f]imidazo[1,2]-d] [1,4] oxazepin-9-yl)pyrrolidine-2-carboxamide;
31) (S)-1-(2-((S)-3-(4-fluorophenyl)-5-isopropyl-2,4-dioxoimidazolidin-1-yl)-5,6-dihydrobenzo[f]imidazo[1,2] -d] [1,4]

oxazepin-9-yl)pyrrolidine-2-carboxamide;
32) (2S)-1-(2-((5R)-3-cyclopropyl -5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
33) (2S)-1-(2-((5S)-3-cyclopropyl -5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
34) (2S)-1-(2-(3-(2-hydroxyethyl)-5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
35) (2S)-1-(2-(3-(2-fluoroethyl)-5-isopropyl-2,4-dioxoimidazolidin-1-yl)-5,6-dihydrobenzo[f]imidazo[1,2-d] [1,4]oxazepin-9-yl)pyrrolidine-2-carboxamide;
36) (2S)-1-(2-(3-(2-(dimethylamino)ethyl)-5-)isopropyl-2,4-dioxoimidazolidin-1-yl)-5,6-dihydrobenzo[f]imidazo[1,2-d] [1,4]oxazepin-9-yl)pyrrolidine-2-carboxamide;
37) (2S)-1-(2-(3-cyclopropyl -5-isopropyl)-2,4-dioxoimidazolidin-1-yl)-5,6-dihydrobenzo[f]imidazo[1,2-d] [1,4]thiazepin-9-yl)pyrrolidine-2-carboxamide;
38) (2S)-1-(2-(3-(6-fluoropyridin-3-yl)-5-isopropyl-2,4-dioxoimidazolidin-1-yl)-5,6-dihydrobenzo[f]imidazo[1,2 -d] [1,4]oxazepin-9-yl)pyrrolidine-2-carboxamide;
39) (2S)-1-(2-(3-(5-fluoropyridin-3-yl)-5-isopropyl-2,4-dioxoimidazolidinl-yl)-5,6-dihydrobenzo[f]imidazo[1,2] -d] [1,4]oxazepin-9-yl)pyrrolidine-2-carboxamide;
40) (2S)-1-(2-(3-(3-cyano-5-fluorophenyl)-5-isopropyl-2,4-dioxoimidazolidin-1-yl)-5,6-dihydrobenzo[f]imidazo[1,2 -d] [1,4]oxazepin-9-yl)-4-fluorinepyrrolidine-2-carboxamide;
41) (2S)-1-(2-(3-(3-chloropyrimidin-2-yl)-5-isopropyl-2,4-dioxoimidazolidin-1-yl)-5,6-dihydrobenzo[f]imidazo[ 1,2 -d] [1,4]oxazepin-9-yl)pyrrolidine-2-carboxamide;
42) (2S)-1-(2-(3-cyclopentyl-5-isopropyl-2,4-dioxoimidazolidin-1-yl)-5,6-dihydrobenzo[f]imidazo[1,2-d] [1,4] oxazepin-9-yl)pyrrolidine-2-carboxamide;
43) (2S)-1-(2-(3-(cyanomethyl)-5-isopropyl-2,4-dioxoimidazolidin-1-yl)-5,6-dihydrobenzo[f]imidazo[1,2-d] [1,4]oxazepin-9-yl)pyrrolidine-2-carboxamide;
44) (2S)-1-(2-(5-isopropyl-3-methyl-2,4-dioxoimidazolidin-1-yl)-5,6-dihydrobenzo[f]imidazo[1,2-d] [1,4] thiazepin-9-yl)pyrrolidine-2-carboxamide;
45) (2S)-1-(2-(3-(2,2-difluoroethyl)-5-isopropyl-2,4-dioxoimidazolidin-1-yl)-5,6-dihydrobenzo[f]imidazo[1,2-d ] [1,4] thiazepin-9-yl)pyrrolidine-2-carboxamide;
46) (2S)-1-(2-(3-cyclopropyl -5-isopropyl-2,4-dioxoimidazolidin-1-yl)-3-vinyl-5,6-dihydrobenzo[f]imidazo[1,2-d] [ 1,4]oxazepin-9-yl)pyrrolidine-2-carboxamide;
47) (2S)-1-(3-chloro-2-(3-cyclopropyl -5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
48) (2S)-1-(2-(3-cyclopropyl -5-(difluoromethyl)-2,4-dioxoimidazolidin-1-yl)-5,6-dihydrobenzo[f]imidazo[1,2-d] [1, 4] thiazepin-9-yl)pyrrolidine-2-carboxamide;
49) (2S,3S)-1-(2-(3-cyclopropyl -5-isopropyl-2,4-dioxoimidazolidin-1-yl)-5,6-dihydrobenzo[f]imidazo[1,2-d] [1,4]oxazepin-9-yl)-3-hydroxypyrrolidine-2-carboxamide;
50) (2S)-1-(2-(3-cyclopropyl -5-isopropyl-2,4-dioxoimidazolidin-1-yl)-3-methyl-5,6-dihydrobenzo[f]imidazo[1,2-d] [ 1,4]oxazepin-9-yl)-4-oxopyrrolidine-2-carboxamide;
51) (2S)-4-cyclohexyl-1-(2-(3-cyclopropyl -5-isopropyl-2,4-dioxoimidazolidin-1-yl)-5,6-dihydrobenzo[f]imidazo[1,2-d] [1,4]oxazepin-9-yl)pyrrolidine-2-carboxamide;
52) (2S)-1-(2-(3-cyclopropyl -5-isopropyl-2, 4-dioxoimidazolidin-1-yl)-5, 6-dihydrobenzo[f]imidazo [1,2-d] [1, 4] thiazepin-9-yl)-5-oxopyrrolidine-2-carboxamide.

The presen invention also provides a pharmaceutical composition, wherein the pharmaceutical composition comprises the therapeutically effective amount of at least one compound of Formula (I) and at least one pharmaceutical acceptable excipient.

The present invention further provides a pharmaceutical composition, wherein the said compound of Formula (I) in the composition has a weight ration about 0.0001-10 with the said excipient.

The present invention provides use of the compound of Formula (I) or the pharmaceutical composition comprising the compound of Formula (I) for the preparation of a medicament.

The present invention further provides the preferred technical solutions about the said use:
Preferably, the medicament is used for the treatment, prevention, delaying or arrestinig onset or progression in cancer or cancer metastasis.

Preferably, the medicament is used for the treatment of cancer.

Preferably, the medicament is used as PI3K inhibitor.

Preferably, the medicament is used for the treatment of the diease mediated by PI3K.

Preferably, the PI3K comprises PI3K$\alpha$, PI3K$\beta$, PI3K$\delta$ and/or PI3K$\gamma$.

Preferably, the PI3K is PI3Kα.

Preferably, the diease mediated by PI3K is cancer.

Preferably, the cancer is selected from sarcoma, prostate cancer, breast cancer, pancreatic cancer, gastrointestinal cancer, colorectal cancer, thyroid cancer, liver cancer, adrenal cancer, glioma,endometrial cancer, melanoma, kidney cancer, bladder cancer, uterine cancer, vaginacancer, ovarian cancer, multiple myeloma, esophageal cancer, leukemia, brain cancer, oral and pharyngeal cancer, laryngeal cancer, lymphoma, basal cell carcinoma, polycythemia vera, essential thrombocythemia.

The present invention also provides a method for treating or preventing disease mediated by PI3K, the said method comprising administering to a subject in need thereof, a therapeutically effective amount of a compound of Formula (I) or a pharmaceutical composition comprising the compound of Formula (I).

Preferably, in the above method, the said PI3K includes PI3Kα, PI3Kβ, PI3Kδ and /or PI3Kγ.

Preferably, in the above method, the said PI3K is PI3Kα.

Preferably, in the above method, the said diease mediated by PI3K is cancer.

Preferably, in the above method, the said cancer is sarcoma, prostate cancer, breast cancer, pancreatic cancer, gastrointestinal cancer, colorectal cancer, thyroid cancer, liver cancer, adrenal cancer, glioma,endometrial cancer, melanoma, kidney cancer, bladder cancer, uterine cancer, vaginacancer, ovarian cancer, multiple myeloma, esophageal cancer, leukemia, brain cancer, oral and pharyngeal cancer,laryngeal cancer, lymphoma, basal cell carcinoma, polycythemia vera, essential thrombocythemia.

The present invention also provides a method for treating cancer, the said method comprising administering to the subject in need thereof a therapeutically effective amount of at least one compound of Formula (I) or a pharmaceutical composition comprising the compound of Formula (I), the said cancer is sarcoma, prostate cancer, breast cancer, pancreatic cancer, gastrointestinal cancer, colorectal cancer, thyroid cancer, liver cancer, adrenal cancer, glioma,endometrial cancer, melanoma, kidney cancer, bladder cancer, uterine cancer, vaginacancer, ovarian cancer, multiple myeloma, esophageal cancer, leukemia, brain cancer, oral and pharyngeal cancer,laryngeal cancer, lymphoma, basal cell carcinoma, polycythemia vera, essential thrombocythemia.

Preferably, in the above method, the subject is human.

The present invention relates to the compound used as PI3K inhibitor and the manufacture of a medicament comprising said compound for the treatment and prevention of diease mediated by PI3K.The said compound act as an active ingredient has characteristics of good therapeutical effect, high selectivity and high bioavailability. As a drug to be marketed soon, the compound has the characteristics of low cost and convenient taking, which is more beneficial to the wide applicaiton of the medicament and more effectively help the subject in need to overcome the pain and improve their life quality.

The terms used in the present invention have the following meanings:

As used herein, unless otherwise indicated, the term "alkyl" includes saturated monovalent hydrocarbon radicals having straight, branched or cyclic moieties. For example, alkyl include but not limited to methyl, ethyl, propyl, isopropyl, cyclopropyl, N-butyl, isobutyl, sec-butyl, tertbutyl, cyclobutyl, cyclopentyl and cyclohexyl. Similary, $C_{1-4}$, as in $C_{1-4}$ alkyl is defined to identify the group as having 1, 2, 3, or 4 carbon atoms in a linear,branched or a cyclic arrangement.

The term "cycloalkyl" refers to a cyclic saturated monovalent alkyl chain. Similary, $C_{3-6}$, as in $C_{3-6}$ cycloalkyl is defined to identify the group as having 3, 4, 5 or 6 carbon atoms in a cyclic saturated monovalent alkyl chain.The typical cycloalkyl include but not limited to cyclopropane, cyclobutane, cyclopentane or cyclohexane and the like.

As used herein, unless otherwise indicated, the term "heterocyclyl" refers to unsubstituted or substituted non aromatic 3 to 6 member monocyclic systems comprising carbon atoms and 1-3 atoms selected from N, O or S. Wherein the nitrogen or sulfur heteroatoms may optionally be oxidized, and the nitrogen heteroatom may optionally be quaternized. The heterocyclyl group may be attached at any heteroatom or carbon atom which results in the creation of a stable structure. The typical heterocyclyl include but not limited to azetidinyl, pyrrolidinyl, piperidinyl, piperazinyl, oxopiperazinyl, oxopiperidinyl, oxoazepinyl, azepinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiamorpholinyl sulfoxide, thiamorpholinyl sulfone and oxadiazolyl.

The term "halogen" refers to fluorine (F), chlorine (Cl), bromine (Br) or iodine (I). Preferablely, halogen refers to fluorine, chlorine and bromine.

The term "halo" refers to fluoro-, chloro-, bromo- or iodo- group.

The term "substituted" refers to a group in which one or more hydrogen atoms are each independently replaced with the same or different substituent(s). Typical substituents include, but are not limited to, halogen, amino, hydroxyl, cyano, alkyl, alkenyl, alkynyl, cycloalkyl, haloalkyl, alkoxy, aryl, haloaryl, arylalkyl, arylalkenyl, heterocyclyl, cycloalkoxy, alkylamino.

As used herein, unless otherwise indicated, the term "aryl" refers to an unsubstituted or substituted mono- or polycyclic ring system containing carbon ring atoms. Preferred aryl is phenyl.

As used herein, unless otherwise indicated, the term "heteroaryl", represents an unsubstituted or substituted

stable five to six membered monocyclic aromatic ring system or an unsubstituted or substituted nine to ten membered fused heteroaromatic ring system or bicyclic heteroaromatic ring system which consists of carbon atoms and one to four heteroatoms selected from N, O or S, and wherein the nitrogen or sulfur heteroatoms may optionally be oxidized, and the nitrogen heteroatom may optionally be quaternized. The heteroaryl group may be attached at any heteroatom or carbon atom which results in the creation of a stable structure. Examples of heteroaryl groups include, but are not limited to thienyl, furanyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzothiadiazolyl, benzotriazolyl adeninyl, quinolinyl or isoquinolinyl.

The term "compound" used in the present invention include the compound shown in Formula (I), and the pharmaceutical acceptable forms thereof, which include the salt, solvent, non-covalent complex, chelate, tereoisomers (including diastereomers, enantiomers and racemates), geometric isomers, isotopically labeled compounds, tautomers, prodrug thereof, or the any mixture of the above compounds.

The term "enantiomer" refers to a pair of stereoisomers that are non-superimposable and mirror images of each other, and a 1:1 mixture of a pair of enantiomers is a "racemic" mixture. When specifying the stereochemistry of the compounds of the present invention, the conventional RS system is used. (For example (1S, 2S) designates a single stereoisomer of known relative and absolute configuration with two chiral centers).

The term "diastereomers" are stereoisomers that have at least two asymmetric atoms, but which are not mirror images of each other. When the compounds are pure enantiomers, the stereochemistry at each chiral carbon can be specified by R or S.

The isolated compound of unknown absolute configuration can be named (+) or (-) according to the direction in which they rotate plane polarized light (right or left) at the wavelength of the sodium D line. Or the isolated compound can be defined according to the retention times of the corresponding enantiomers/diastereoisomers for chiral HPLC.

Those skilled in the art will recognize that the compounds of the present invention contain chiral centers and thus may exist in different isomeric formulas. Unless otherwise indicated, the compounds of the present invention are intended to include all such possible isomers, including racemic mixture, optically pure formula and mixture of isomers in any ratio. For the compound of example Formula (VIII), including the compound of example 2, the compound of example 32, the compound of example 33, and any ratio of mixtures of example 32 and Example 33. Optically active (R)- and (S)-isomers can be prepared synthetically using optically active starting materials or prepared using chiral reagents, or resolved using conventional techniques (For example, separated on a chiral SFC or HPLC column).

The "pharmaceutically acceptable" refers to those known to be used in animals, especially in human.

The term "composition", as used herein, is intended to encompass a product comprising the specified ingredients in the specified amounts, as well as any product which results, directly or indirectly, from combinations of the specified ingredients in the specified amounts. Accordingly, pharmaceutical compositions containing the compounds of the present invention as the active ingredient as well as methods of preparing the instant compounds are also part of the present invention. Furthermore, some of the crystalline forms for the compounds may exist as polymorphs and as such are intended to be included in the present invention. In addition, some of the compounds may form solvates with water (i.e., hydrates) or common organic solvents and such solvates are also intended to be encompassed within the scope of this invention.

The term "therapeutically effective amount" in the present invention refers to a amount of the compound that is active when it is used to treat and prevent or inhibit at least one clinical symptom of a disease, condition, symptom, indication and/or discomfort.The specific "therapeutically effective amount" will vary since the compound, route of administration, patient age, patient weight, type of disease or discomfort being treated, symptoms and severity are different. In any case, an appropriate dosage will be obvious to those skilled in the art and can be measured by the conventional experiment.

The compounds of the present invention may also be present in the form of pharmaceutically acceptable salts. For use in medicine, the salts of the compounds of this invention refer to non-toxic "pharmaceutically acceptable salts". The pharmaceutically acceptable salt forms include pharmaceutically acceptable acidic/anionic or basic/cationic salts. The pharmaceutically acceptable acidic/anionic salt generally takes a form in which the basic nitrogen is protonated with an inorganic or organic acid. Representative organic or inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic. Pharmaceutically acceptable basic/cationic salts include, and are not limited to aluminum, calcium, chloroprocaine, choline, diethanolamine, ethylenediamine, lithium, magnesium, potassium, sodium and zinc.

The prodrugs of the compounds are included in the scope of the present invention. In general, such prodrugs will be functional derivatives of the compounds that are readily converted in vivo into the required compound. Thus, in t he methods of treatment of the present invention, the term "administering" shall encompass the treatment of the various disorders described with the compound specifically disclosed or with a compound which may not be specifically disclosed,

but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

When the compound of Formula I and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and ect. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N',N'- dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, formic, hydrochloric, maleic, phosphoric, sulfuric and tartaric acids, particularly preferred are formic and hydrochloric acid. Since the compounds of Formula I are intended for pharmaceutical use they are preferably provided in substantially pure form, for example at least 60% pure, more suitably at least 75% pure, especially at least 98% pure (% are on a weight for weight basis).

The pharmaceutical compositions of the present invention comprise a compound represented by Formula I (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula I, or a prodrug, or a metabolite, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as oil-in-water emulsion, or as a water-in- oil liquid emulsion. In addition to the common dosage forms set out above, the compound represented by Formula I, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound, or a pharmaceutically acceptable salt, of Formula I. The compounds of Formula I, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include such as lactose, terra alba, sucrose, talc, gelatin, agar, pectin, acacia, magnesium stearate, and stearic acid. Examples of liquid carriers include such as sugar syrup, peanut oil, olive oil, and water. Examples of gaseous carriers include such as carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1 mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

## Examples

The present invention will use the following examples to illustrate the preparation of the compound of Formula (I), but there's no limit to the present invention.

The following examples are offered for illustrative purposes, so that those skilled in the art can understand the present invention, but are not intended to limit the invention in any manner. Unless otherwise indicated, the technical solutions or the methods of the examples in the present inventions are conventional. Unless otherwise indicated, all parts and percentages are by weight, all temperatures are in degrees Celsius of the present invention.

The following abbreviations have been used in the examples:

DCM: Dichloromethane
DMF: N, N-dimethylcarboxamide
DIEA: N, N-diisopropylethylamine
PE: Petroleum ether
EA: Ethyl acetate
NIS: N-Iodosuccinimide
LCMS or LC-MS: Liquid chromatography-mass spectrometry
THF: Tetrahydrofuran
DMSO: Dimethyl sulfoxide
$Et_3N$ or TEA: Triethylamine
HATU: 2-(7-Azobenzotriazole)-N, N, N', N'-tetramethylurea hexafluorophosphate Hex: n-hexane
h, hr or hrs: hour
LiHMDS: Lithium bis (trimethylsilyl) amide
$[PdCl_2(dppf)]CH_2Cl_2$: [1, 1'-bis (diphenylphosphine) ferrocene] dichloropalladium (II), complex with dichloromethane
Boc: Tert-Butoxycarbonyl
min: minute
rt or RT: room temperature.

**General route:**

Compound of Formula (VI) was prepared by the following route:

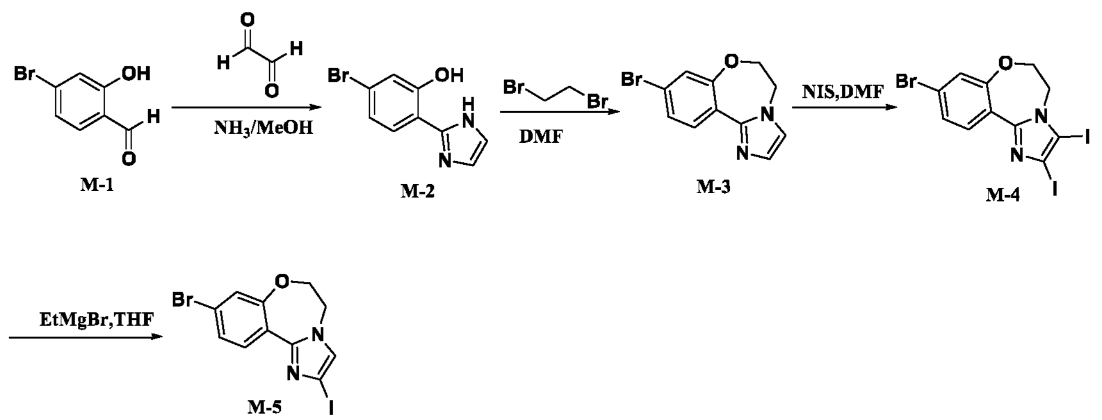


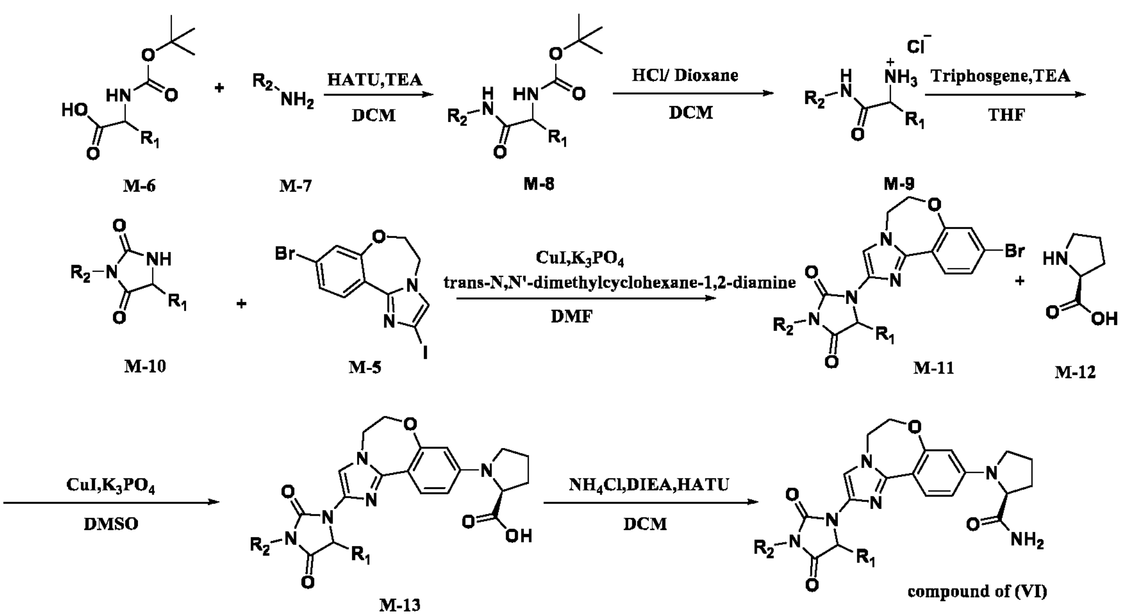

**Preparation of intermediate M-5**:

**Step 1: Synthesis of compound M-2**

4-bromo-2-hydroxybenzaldehyde (40 g) and MeOH (400 mL) were added into a 1000 mL single-neck flask, ammonia (136.50 g) was added with stirring in an ice bath, and then reacted in oil bath at 35 °C. When LC-MS analysis indicated completion of the reaction, the reaction mixture was concentrated and diluted with water, extracted with EA for four times, and then the organic layers were combined, dried and concentrated. The residue was purified by column chromatography (PE: EA=3:1), and concentrated to give compound M-2 (34.55 g).
LC-MS [$M+H^+$]: 239.

**Step 2: Synthesis of compound M-3**

Compound M-2 (34.55 g), $Cs_2CO_3$ (133 g) and DMF (300 mL) were added into a 1000 mL single-neck flask, stirred at room temperature for 20 mins and then 1,2-dibromoethane (54.30 g) was added dropwised, after the addition was finished, the mixture was put into a 80°C oil bath for a reflux reaction. When LC-MS analysis indicated the completion of the reaction, the reaction mixture was concentrated and diluted with water, extracted with EA for 3 times, and then the organic layers were combined, dried, concentrated under reduced pressure.The residue was purified by column chromatography (PE: EA=70:30), concentrated to give compound M-3 (21.37 g).
LC-MS [$M+H^+$]: 265.
$^1H$ NMR (500 MHz, Chloroform-d) $\delta$ 8.38 (d, J = 8.5 Hz, 1H), 7.30-7.15 (m, 3H), 6.99 (s, 1H), 4.47-4.43 (m, 2H), 4.41 -4.35 (m, 2H).

**Step 3: Synthesis of compound M-4**

Compound M-3 (21.37 g) and DMF (100 mL) were added into a 1000 mL single-neck flask, stirred to dissolve, and NIS (50.78 g) dissolved in DMF (100 mL) was added dropwised, after the addition was finished, the reaction was put into a 60°C oil bath and reacted overnight. When LC-MS analysis indicated the completion of the reaction, water was added into the reaction mixture, the resulting precipitate was collected by vacuum filtration, and the filter cake was dried to give compound M-4 (35 g).
LC-MS [$M+H^+$]: 517.
$^1H$ NMR (500 MHz, Chloroform-d) $\delta$ 8.30 (d, J = 8.6 Hz, 1H), 7.25-7.16 (m, 2H), 4.46 -4.41 (m, 2H), 4.36-4.32 (m, 2H).

**Step 4: Synthesis of compound M-5**

Compound M-4 (35 g) and THF (150 mL) were added into a 500mL three-neck flask, 100 mL ethylmagnesium bromide (1 M THF solution) was added dropwised at -40°C under nitrogen protection, after the addition was finished, the reaction was stirred at -40°C. When LC-MS analysis indicated the completion of the reaction, the reaction mixture was quenched with saturated ammonium chloride solutionin in an ice bath, extracted with EA for 3 times, and then the organic layers were combined, dried, concentrated to provide a residue, the residue was slurried in methyltertbutyl ether, the suspension was filtered off with suction and the filter cake was dried to give compound M-5(19.8 g).
LC-MS [$M+H^+$]: 391.
$^1H$ NMR (500 MHz, DMSO-d6) $\delta$ 8.22 (d, J = 8.6 Hz, 1H), 7.55 (s, 1H), 7.31 -7.23 (m, 2H), 4.47 -4.39 (m, 4H).

**Example 1 Synthesis of (2S)-1-(2-(5-isopropyl-3-methyl-2,4-dioxoimidazolidin-1-yl)-5, 6-dihydrobenzo[f] imidazo [1, 2-d] [1,4]oxazepin-9-yl)pyrrolidine-2-carboxamide (compound 1)**

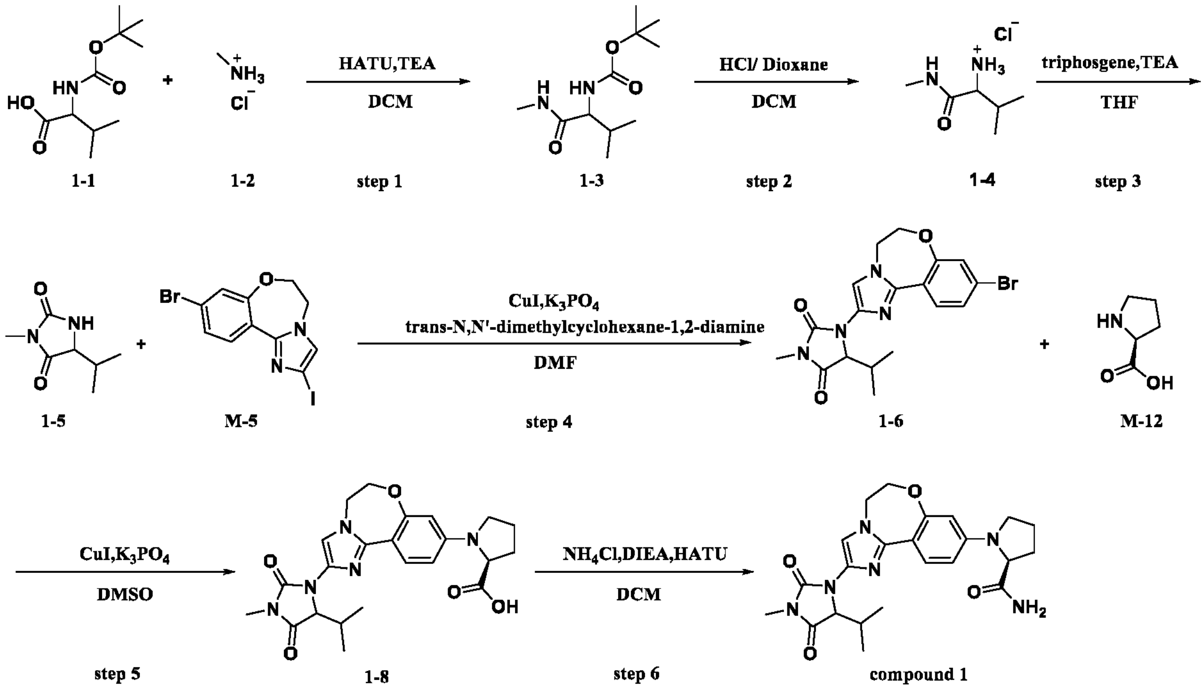


**Step 1: Synthesis of compound 1-3**

Compound 1-1 (9 g), compound 1-2 (16.8 g), DCM (200 mL) and HATU (31.25 g) were added into a 500mL three-neck flask, TEA(50.21 g) was added dropwised in an ice bath, after the addition was finshed, reacted at room temperature. When LC-MS analysis indicated the completion of the reaction, quenched the reaction with the addition of water, the organic layers were separated, rinsed with water, dried over anhydrous $Na_2SO_4$ and concentrated. The residue was purified by column chromatography (PE: EA=50:50) to give compound 1-3 (9.2g).

LC-MS [M-Boc+H] $^+$: 131.

**Step 2: Synthesis of compound 1-4**

HCl/Dioxane (10mL, 4.0mol/L) was added to the solution of compound 1-3 (1.3g) in dichloromethane (10mL), stirred at room temperature for 3 hours.After the reaction was completed, the reaction mixture was concentrated to give compound 1-4 (0.9 g), which was used in the next reaction directly without further purification.

LC-MS [M+H] $^+$: 131.

**Step 3: Synthesis of compound 1-5**

Compound 1-4 (900 mg) and TEA (4.19 g) were dissolved in THF (50 mL). Triphosgene (802 mg) in THF (10 mL) was added slowly to the reaction in an ice bath, the reaction mixture was naturally heated to room temperature and stirred for 12h. When LC-MS analysis indicated the completion of the reaction, the reaction was quenched with the addition of water (5 mL), the reaction mixture was concentrated, extracted and separated with dichloromethane/water. The organic layers were dried over anhydrous $Na_2SO_4$ and concentrated. The residue was purified by column chromatography (PE: EA=1:1) to give compound 1-5 (732 mg).

LC-MS [M+H] $^+$: 157.

**Step 4: Synthesis of compound 1-6**

Compound 1-5 (177 mg), compound M-5 (443 mg), CuI (64.75 mg), trans-N,N'-dimethylcyclohexane-1,2-diamine (48.36 mg) and $K_3PO_4$ (721.68 mg) were dissolved in DMF(5 mL), the atmosphere was replaced with nitrogen for three times, the reaction mixture was heated to 110 °C and reacted for 2 h. When LC-MS analysis indicated the completion of the reaction, the reaction mixture was diluted with EA (100 mL), washed with water. The organic layers were combined, dried over anhydrous $Na_2SO_4$ and concentrated. The residue was purified by column chromatography (PE: EA=70:30) to give compound1-6 (350 mg).

LC-MS [M+H] $^+$: 419.

**Step 5: Synthesis of compound 1-7**

Compound 1-6 (350 mg), L-proline (290 mg), $K_3PO_4$ (848 mg), CuI (79.8 mg) and DMSO (5 mL) were added to a 100 mL single-neck flask, the reaction was stirred at 120 °C for 3 h under nitrogen protection. When LC-MS analysis indicated the completion of the reaction, the reaction mixture was filtered, the filter cake was washed with DMSO (3 mL), the filtrate was used in the next step directly.

LC-MS $[M+H]^+$: 454.

Step 6: **Synthesis of compound 1**

DCM (12 mL), $NH_4Cl$ (445 mg) and DIEA (2.17 g) were added to the filtrate in Step 5 under nitrogen protection, cooled to 0 °C, HATU (1.27 g) was added to the reaction system in an ice bath, stirred at 0 °C for 20 mins. When LC-MS analysis indicated the completion of the reaction, the reaction mixture was diluted with DCM, washed with water, the organic layers was dried over anhydrous $Na_2SO_4$ and concentrated. The resulting crude product was purified by column chromatography (PE: EA=100:0-0:100) to give compound 1 (300mg).

LC-MS $[M+H]^+$: 453.

$^1H$ NMR (500 MHz, DMSO-d6) $\delta$ 8.04 (d, J = 8.9 Hz, 1H), 7.41 (s, 1H), 7.24 (s, 1H), 7.05 (s, 1H), 6.32 (d, J = 8.7 Hz, 1H), 6.03 (s, 1H), 4.62-4.51 (m, 1H), 4.45 -4.30 (m, 4H), 3.94 (d, J = 8.7 Hz, 1H), 3.55 (t, J = 7.3 Hz, 1H), 3.23 (q, J = 7.2 Hz, 1H),2.92 (s, 3H), 2.68 (m, 1H), 2.26-2.12 (m, 1H), 1.96 (m, 3H), 1.16 (d, J = 7.0 Hz, 3H), 0.74 (d, J = 6.8 Hz, 3H).

**Example 2 Synthesis of (2S)-1-(2-(3-cyclopropyl-5-isopropyl-2,4-dioxoimidazolidin-1-yl)-5,6-dihydrobenzo[f]imidazo[1,2-d][1,4]oxazepin-9-yl)pyrrolidine-2-carboxamide(compound 2)**

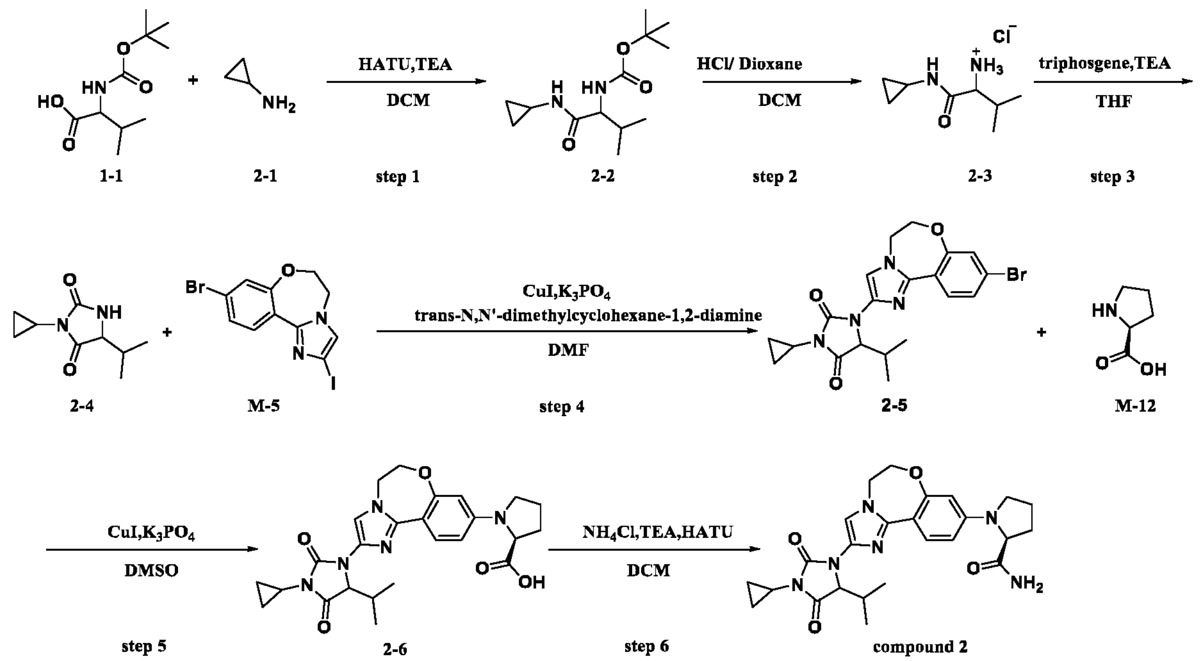


**Step 1: Synthesis of compound 2-2**

Compound1-1 (5.0 g), DCM (100 mL) and HATU (9.55 g) were added to a 250 mL single-neck flask under nitrogen protection, TEA (8.15 g) and compound 2-1(1.45 g) were added to the reaction in an ice bath, reacted at room tempreture for 2h. When LC-MS analysis indicated the completion of the reaction, the reaction mixture was concentrated, diluted with EA, washed with water, dried over anhydrous $Na_2SO_4$ and concentrated to give compound 2-2 (5.0 g), which was used in the next step directly.

LC-MS $[M-Boc+H]^+$: 157.

$^1H$ NMR (500 MHz, DMSO-d6) $\delta$ 7.91 (s, 1H), 6.54 (d, J=9.0 Hz, 1H), 3.69-3.58 (m, 1H), 2.65-2.55 (m, 1H), 1.88-1.80 (m, 1H), 1.37 (s, 9H), 0.79 (d, J=6.0 Hz, 6H), 0.62-0.58 (m, 2H), 0.42-0.30 (m, 2H).

**Step 2: Synthesis of compound 2-3**

Compound 2-2 (5 g), DCM (20 mL) and HCl/Dioxane (20 mL, 4.0mol/L) were added to a 250 mL single-neck flask, and reacted at room tempreture for 2 h. When LC-MS analysis indicated the completion of the reaction, the reaction mixture was concentrated to give compound 2-3 (3.75 g).

LC-MS [M +H] $^+$: 157.

**Step 3: Synthesis of compound 2-4**

Compound 2-3 (2.0 g), DCM (50 mL) and TEA (4.20 g) were added to a 250 mL single-neck flask, under nitrogen protection, triphosgene (1.54 g) dissolved in DCM (50 mL) was added to the reaction system in an ice bath, the mixture was stirred in an ice bath for 2 h. When LC-MS analysis indicated the completion of the reaction, the reaction was quenched with ice water in an ice bath, the reaction mixture was concentrated, extracted with EA, dried over anhydrous $Na_2SO_4$, concentrated to give a crude product. The crude product was purified by column chromatography (PE: EA=100:0-50:50), to give compound 2-4 (810mg).

LC-MS [M+H] $^+$: 183/185.

$^1$H NMR (500 MHz, Chloroform-d) δ 6.28 (s, 1H), 3.85 (d, J = 3.6 Hz, 1H), 2.66 - 2.48 (m, J = 3.7 Hz, 1H), 2.25-2.15 (m, 1H), 1.03 (d, J = 6.5 Hz, 3H), 0.98 -0.91 (m, 4H), 0.88 (d, J = 7.0 Hz, 3H).

**Step 4: Synthesis of compound 2-5**

Compound 2-4 (431 mg), compound M-5 (700 mg), DMF (10 mL), CuI (102 mg), trans-N,N'-dimethylcyclohexane-1,2-diamine (77 mg) and $K_3PO_4$ (760 mg) were added to a 50 mL single-neck flask, under nitrogen protection, the reaction mixture was heated to 120 °C and stirred for 2h. When LC-MS analysis indicated the completion of the reaction, the reaction mixture was diluted with EA, washed with water, dried over anhydrous $Na_2SO_4$ and concentrated. The crude product was purified by column chromatography (PE: EA=100:0-60:40) to give compound 2-5 (642mg).

LC-MS [M+H] $^+$: 445/447.

$^1$H NMR (500 MHz, Chloroform-d) δ 8.23 (d, J = 8.5 Hz, 1H), 7.27 (s, 1H), 7.22 (d, J = 8.7 Hz, 1H), 7.20 (s, 1H), 4.63-4.59 (m, 1H), 4.51-4.38 (m, 2H), 4.36 (t, J = 4.3 Hz, 2H), 2.79-2.71 (m, 1H), 2.68-2.60 (m, 1H), 1.24 (d, J = 7.1 Hz, 3H), 1.02-0.94 (m, 4H), 0.81 (d, J = 6.9 Hz, 3H).

**Step 5: Synthesis of compound 2-6**

Compound 2-5 (642 mg), compound M-12 (415 mg), $K_3PO_4$ (919 mg) and DMSO (10 mL) were added to 30 mL microwave vial, purged with nitrogen, CuI (83 mg) was added, the reaction mixture was heated to 120 °C and reacted in a microwave reactor for 1 h. When LC-MS analysis indicated the completion of the reaction, the reaction mixture was used in the next step directly.

LC-MS [M+H] $^+$: 480.

**Step 6: Synthesis of compound 2**

The reaction mixture in Step 5 was added to a 50 mL single-neck flask, the atmoshpere was replaced with nitrogen, DCM (10 mL), $NH_4Cl$ (462 mg) and TEA (1.46 g) were added, the reaction system was cooled to 0 °C, HATU (3.28 g) was added in an ice bath, the reaction mixture was stirred at 0 °C for 1 h. When LC-MS analysis indicated the completion of the reaction, the reaction mixture was diluted with DCM, washed with water, the organic layers were dried over anhydrous $Na_2SO_4$ and concentrated. The crude product was purified by Pre-HPLC (C18 column, $H_2O$: MeOH=95:5-50:50), to give compound 2 (85mg).

LC-MS [M+H]$^+$: 479.

$^1$H NMR (500 MHz, DMSO-d6) δ 8.03 (d, J = 9.0 Hz, 1H), 7.40 (s, 1H), 7.24 (s, 1H), 7.05 (s, 1H), 6.32 (d, J = 8.9 Hz, 1H), 6.03 (d, J = 2.6 Hz, 1H), 4.48 (s, 1H), 4.41 -4.29 (m, 4H), 3.94 (d, J = 8.8 Hz, 1H), 3.55 (t, J = 8.1 Hz, 1H), 3.25-3.19 (m, 1H), 2.74 -2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

**Step 7: Preparation of compound 32 and compound 33**

In the present examples, compound 32 (front peak) and compound 33(back peak) were prepared by the separation of the compound 2 through the the following chiral column.

Chiral column HPLC conditions:

| Column | CHIRALPAK IA |
|---|---|
| Column Specifications | 3cm×25cm,5um |
| Injection volume | 4.0mL |
| Mobile phase | (Hex:DCM =3:1):EtOH=50:50 (v/v) |
| Flow rates | 35mL/min |
| Wavelength | UV 220 nm |
| Temperature | 25°C |
| Sample solution | EtOH:DCM=3:1(12.3mg/mL) |
| Pre-HPLC | Prep-HPLC-flash |

Compound 32: LC-MS [M+H]: 479.

$^1$H NMR (500 MHz, DMSO-d6) δ 8.03 (d, J = 9.0 Hz, 1H), 7.40 (s, 1H), 7.24 (s, 1H), 7.05 (s, 1H), 6.32 (d, J = 8.9 Hz, 1H), 6.03 (d, J = 2.6 Hz, 1H), 4.48 (s, 1H), 4.41 - 4.29 (m, 4H), 3.94 (d, J = 8.8 Hz, 1H), 3.55 (t, J = 8.1 Hz, 1H), 3.25-3.19 (m, 1H), 2.74 - 2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

Compound 33: LC-MS $[M+H]^+$: 479.

$^1$H NMR (500 MHz, DMSO-d6) δ 8.03 (d, J = 9.0 Hz, 1H), 7.40 (s, 1H), 7.24 (s, 1H), 7.05 (s, 1H), 6.32 (d, J = 8.9 Hz, 1H), 6.03 (d, J = 2.6 Hz, 1H), 4.48 (s, 1H), 4.41 - 4.29 (m, 4H), 3.94 (d, J = 8.8 Hz, 1H), 3.55 (t, J = 8.1 Hz, 1H), 3.25-3.19 (m, 1H), 2.74 - 2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

**Example 3 Synthesis** of **(2S)-1-(2-(5-isopropyl-2,4-dioxo-3-(2,2,2-trifluoroethyl)imidazolidine-1-yl)-5,6-dihydrobenzo[f]imdazo[1, 2-d] [1,4]oxazepin-9-yl)pyrrolidine-2-carboxamide(compound 3)**

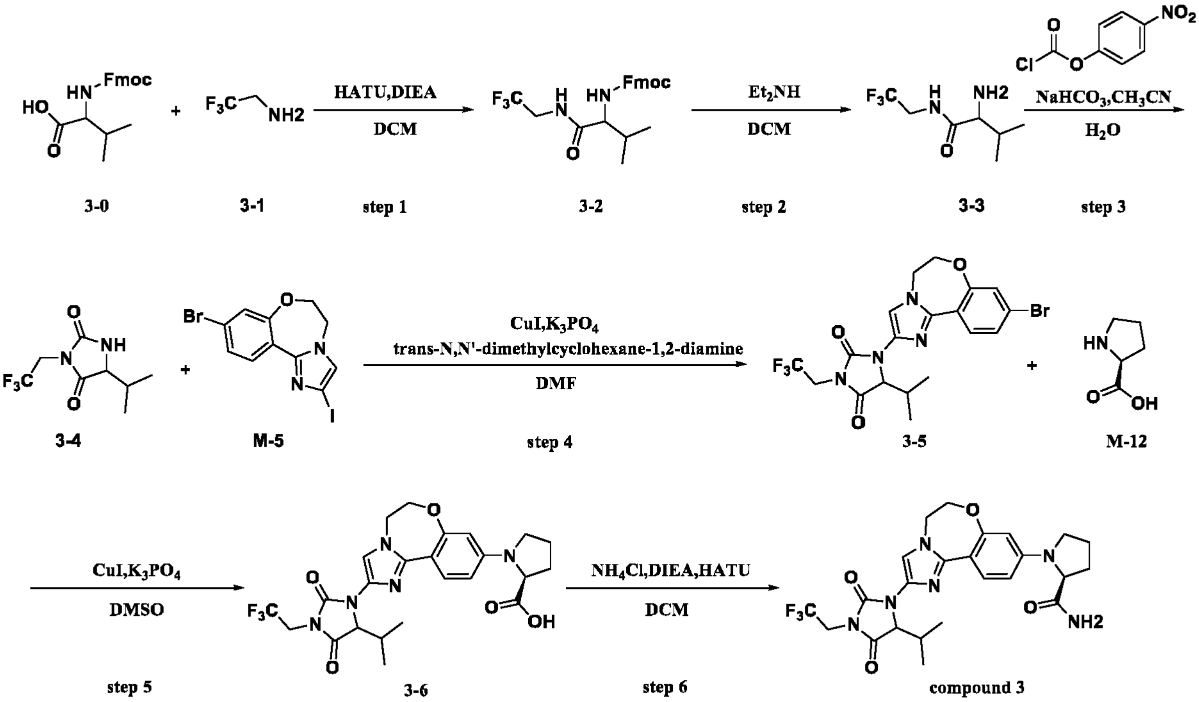


**Step1: Synthesis of compound 3-2**

To a solution of compound 3-0 (2 g) and compound 3-1(583 mg) in DCM (100 ml), HATU (2.67 g) and DIEA

(2.28 g) were added successively, the mixture was stirred at room temperature for 12 h. Water was added, and the reaction mixture was separated, concentrated to give a residue.EA (25 mL) was added to the residue, and then the residue was washed with water and saturated brine. The organic layers were dried over anhydrous $Na_2SO_4$, concentrated, to give 1.8 g product.

LC-MS $[M+H]^+$: 421.

**Step 2: Synthesis of compound 3-3**

Diethylamine (10 mL) was added to the solution of compound 3-2 (1.8 g) dissolved in DCM (10 ml), stirred at room temperature for 2h.The mixture was concentrated under reduced pressure to give a residue, and the residue was purified by column chromatography to give compound 3-3 (0.70 g).

LC-MS $[M+H]^+$: 199.

Step 3: **Synthesis of compound 3-4**

Compound 3-3 (200 mg), $CH_3CN$ (10 mL), $NaHCO_3$ (254 mg) were added to a 50 mL three-neck flask, the atmosphere was replaced with nitrogen for 3 times, p-Nitrophenyl chloroformate (203 mg) was added and the mixture was stirred at room temperature for 2 h. Water (6 mL) was added to the reaction system, and stirred at room temperature for 3h.When the reaction was completed, the reaction mixture was concentrated under reduced pressure to provide the residue, and the residue was diluted with EA, the organic layers were washed with water, potassium carbonate aqueous solution and saturated brine successively, and dried over anhydrous $Na_2SO_4$, concentrated to provide the crude product. The crude product was purified by the column chromatography (PE: EA=60:40) to give compound 3-4 (195 mg).

LC-MS $[M+H]^+$: 225.

**Step 4: Synthesis of compound 3-5**

Compound 3-4 (138 mg), compound M-5 (200 mg), CuI (39 mg), trans-N,N'-dimethylcyclohexane-1,2-diamine (29 mg), $K_3PO_4$ (326 mg) were dissolved in DMF(5 mL), the atmosphere was replaced with nitrogen for 3 times, the reaction mixture was heated to 110 °C for 2 h. When the reaction was completed, the reaction mixture was diluted with EA, washed with water for one time and saturated brine for three times, and the organic layers were dried over anhydrous $Na_2SO_4$ and concentrated. The residue was purified by column chromatography (PE: EA=100:0-70:30) to give compound 3-5 (180 mg).

LC-MS $[M+H^+]$: 487/489.

**Step 5: Synthesis of compound 3-6**

Compound 3-5 (117 mg), compound M-12 (138 mg), $K_3PO_4$ (356 mg), CuI (46 mg) and DMSO (3 mL) were added to a 50 mL single-neck flask, the atmosphere was replaced with nitrogen for 3 times, the reaction mixture was heated to 120 °C and stirred for 3 h. When the reaction was completed, the reaction mixture was filtered, the filter cake was washed with DMSO (3 mL), the filtrate was used in the next step directly.

LC-MS $[M+H]^+$: 522.

**Step 6: Synthesis of compound 3**

DCM (6 mL), $NH_4Cl$ (127 mg), DIEA (613 mg) were added to the filtrate in Step 5 under nitrogen protection, the reaction mixture was cooled to 0 °C, HATU (447 mg) was added in portions in an ice bath, stirred at 0 °C for 20 mins. When the reaction was completed, the reaction mixture was diluted with dichloromethane, washed with water and saturated brine, the organic layers were dried over anhydrous $Na_2SO_4$, concentrated under reduced pressure to provide crude product. The crude product was purified by Pre-HPLC to give compound 3 (85 mg).

LC-MS $[M+H]^+$ : 521.

$^1H$ NMR (500 MHz, DMSO-d6) $\delta$ 8.05 (d, J = 8.5 Hz, 1H), 7.41 (s, 1H), 7.29 (s, 1H), 7.06 (s, 1H), 6.33 (d, J = 9 Hz, 1H), 6.04 (s, 1H), 4.71 - 4.70 (m, 1H), 4.38 - 4.24 (m, 6H), 3.95 (d, J = 8.5 Hz, 1H), 3.55 (t, J = 7.3 Hz, 1H), 3.28-3.20 (m, 1H), 2.70 (brs, 1H), 2.25-2.15(m, 1H), 2.00-1.94(m, 3H), 1.17 (d, J = 7.0 Hz, 3H), 0.75 (d, J = 7 Hz, 3H).

**Example 4 Synthesis of (2S)-1-(2-(3-(4-fluorophenyl)-5-isopropyl-2, 4-dioxoimidazolidin-1-yl)-5, 6-dihydrobenzo[f] imidazo [1, 2-d][1,4]oxazepin-9-yl)pyrrolidine-2-carboxamide(compound 4)**

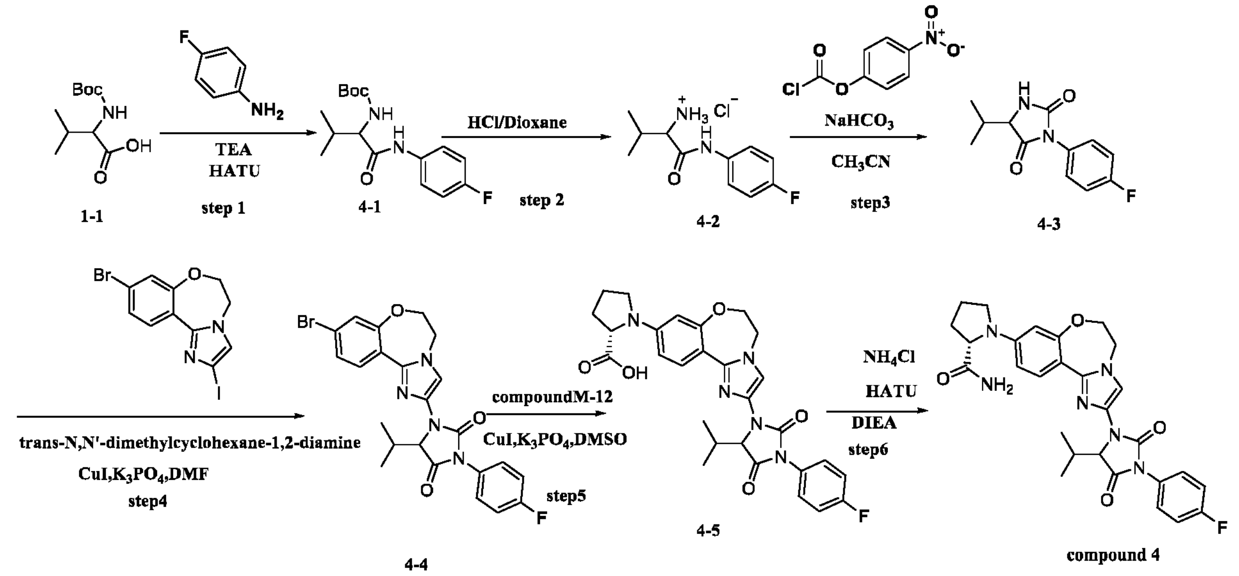


1-1 4-1 4-2 4-3 4-4 4-5 compound 4

### Step 1: Synthesis of compound 4-1

HATU (3.39 g) and DIEA (2.68 g) were added to the solution of compound 1-1 (1.5 g) and p-Fluoroaniline (921 mg) in DCM (50 ml). The reaction mixture was stirred at room temperature for 12 h. When the reaction was completed, the reaction mixture was diluted with water, separated, the organic layers were washed with 1 N HCl, $NaHCO_3$ saturated aqueous solution and saturated brine successively, dried over anhydrous $Na_2SO_4$, concentrated to provide crude product. The crude product was purified by column chromatography (EA/PE =0-30 %) to give compound 4-1 (2.1 g).
LC-MS [M-Boc+H] $^+$: 211.

### Step 2: Synthesis of compound 4-2

Compound 4-1 (2.1 g) was added to the solution of HCl/dioxane (10 mL, 4 M), stirred at room temperature for 12h. The reaction mixture was concentrated under reduced pressure to give compound 4-2 (1.64 g), which was used in the next step directly without the further purification.
LC-MS [M+H] $^+$: 211.

### Step 3: Synthesis of compound 4-3

Compound 4-2 (500 mg), $CH_3CN$ (30 mL), $NaHCO_3$ (681 mg) were added to a 50 mL three-neck flask, the atmosphere was replaced with nitrogen for 3 times, p-Nitrophenyl chloroformate (449 mg) was added, stirred at room tempreture for 2h. Water (18 mL) was added to the reaction mixture, and stirred at room temperature for 3 h.
When the reaction was completed, the reaction mixture was concentrated under reduced pressure to provide the residue, the residue was diluted with EA (100 mL), the organic layers were washed with water (50 mL), 5 % $K_2CO_3$ aqueous solution (50 mL) and saturated brine respectively, seperated, the organic layers were dried over anhyfrous $Na_2SO_4$, concentrated to provide the crude product. The crude product was purified by column chromatography (EA/PE=0-40 %) to give compound 4-3 (447 mg).
LC-MS [M+H] $^+$: 237.

### Step 4: Synthesis of compound 4-4

Compound 4-3 (144 mg), compound M-5 (200 mg), CuI (39 mg), trans-N,N'-dimethylcyclohexane-1,2-diamine (29 mg), $K_3PO_4$ (326 mg) were dissolved in DMF (5 mL), the atmosphere was replaced with nitrogen for 3 times, the reaction mixture was heated to 110 °C and reacted for 2 h.When the reaction was completed, EA(100 mL) was added to dilute the reaction mixture.The organic layers were washed with water (60 mL) and saturated brine(60 mL), and dried over anhydrous $Na_2SO_4$ and concentrated. The residue was purified by column chromatography (PE: EA=100:0-70:30) to give compound 4-4 (215 mg).
LC-MS [M+H]$^+$: 499.

**Step 5: Synthesis of compound 4-5**

4-4 (120 mg), compound M-12 (138 mg), $K_3PO_4$ (356 mg), CuI (46 mg) and DMSO (3mL) were added to a 100 mL single-neck flask, the atmosphere was replaced with nitrogen for 3 times, the reaction mixture was heated to 120 °C, stirred and reacted for 3 h. When the reaction was completed, the reaction mixture was filtered, filter cake was washed with 3 mL of DMSO, the filtrate was used in the next step directly.

LC-MS [M+H$^+$]: 534.

**Step 6: Synthesis of compound 4**

DCM (12mL), $NH_4Cl$ (445 mg) and DIEA (2.17 g) were added to the filtrate in Step5 under nitrogen protection, the reaction mixture was cooled to 0 °C, HATU (1.27 g) was added in portions in an ice bath, and stirred at 0 °C for 20 mins. When the reaction was completed, the reaction minture was diluted with DCM (50 mL), the organic layers were washed with water (40 mL) and saturated brine(40 mL), dried over anhydrous $Na_2SO_4$, concentrated to provide the crude product. The crude product was purified by column chromatography (PE: EA=100:0-0:100) to give compound 4 (89 mg).

LC-MS [M+H]$^+$: 533.

$^1$H NMR (500 MHz, DMSO-$d_6$) δ 8.08 (d, *J* = 8.9 Hz, 1H), 7.45-7.41 (m, 3H), 7.38-7.35 (m, 2H), 7.28 (s, 1H), 7.06 (s, 1H), 6.34 (d, *J*= 8.9 Hz, 1H), 6.04 (s, 1H), 4.76 (s, 1H), 4.40-4.37 (m, 4H), 3.95 (d, *J* = 8.8 Hz, 1H), 3.60-3.52 (m, 1H), 3.24 (q, *J* = 8.5, 7.7 Hz, 1H), 2.75 (s, 1H), 2.26 - 2.14 (m, 1H), 2.00-1.93 (m, 3H), 1.22 (d, *J*= 7.1 Hz, 3H), 0.88 (d, *J*= 6.8 Hz, 3H).

Compound 30 and compound 31 were prepared following similar procedures as described in the Step 7 in Example 2.

**Example 5 Synthesis of (2S)-1-(2-(5-cyclopropyl -3-methyl-2, 4-dioxoimidazolidin-1-yl)-5, 6-dihydrobenzo[f] imidazo [1, 2-d] [1, 4] oxazepin-9-yl) pyrrolidine-2-carboxamide (compound 5)**

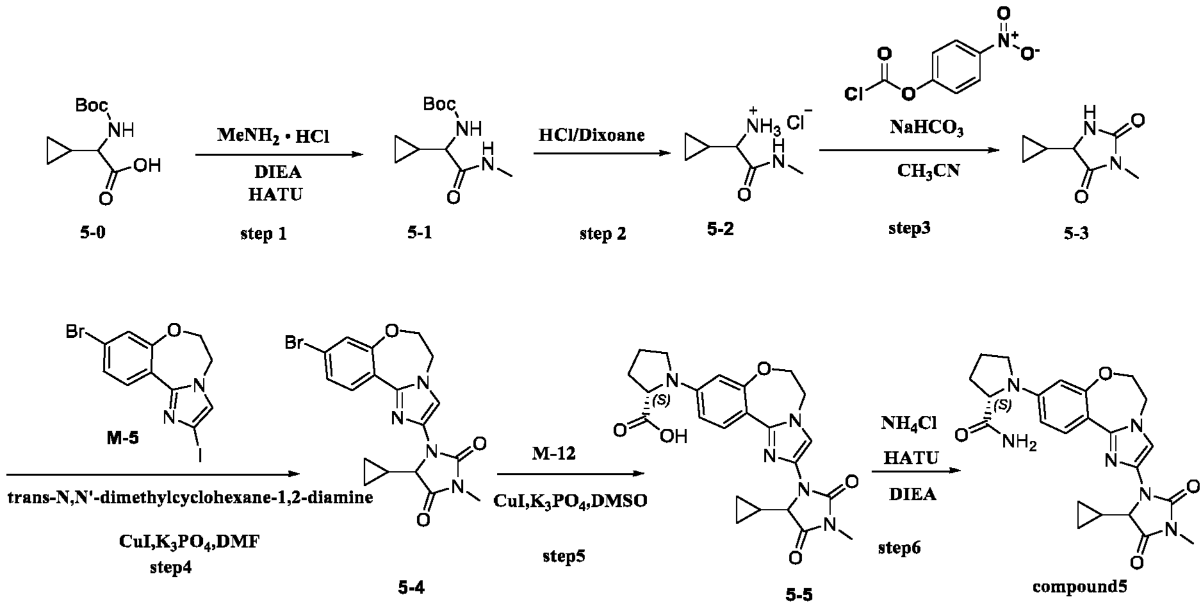


Replacing compound 1-1 with compound 5-0, and p-fluoroaniline with methylamine hydrochloride, compound 5 was prepared following similar procedures as described in Example 4.

LC-MS [M+H] $^+$: 451.

**Example 6 Synthesis of (2S)-1-(2-(3-cyclobutyl-5-isopropyl-2, 4-dioxoimidazolidin-1-yl)-5, 6-dihydrobenzo[f] imidazo [1,2-d] [1,4] oxazepin-9-yl)pyrrolidine-2-carboxamide(compound 6)**

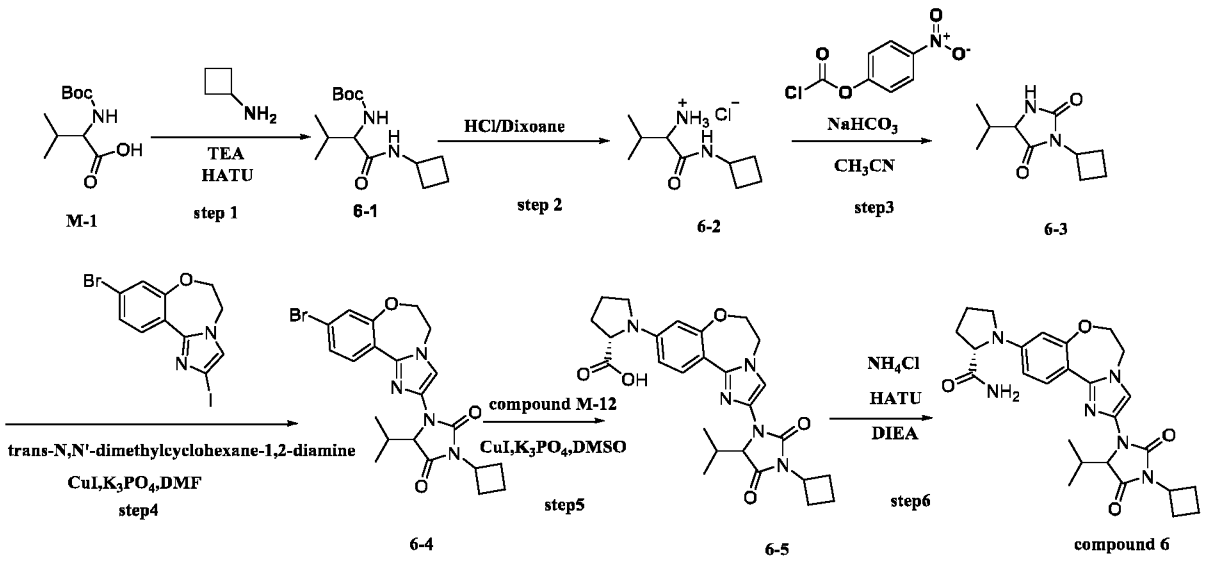


Replacing p-fluoroaniline with cyclobutylamine, compound 6 was prepared following similar procedures as described in Example 4.

LC-MS $[M+H]^+$: 493.

$^1H$ NMR (500 MHz, DMSO-d6) δ 8.04 (d, J = 9 Hz, 1H), 7.41 (s, 1H), 7.25 (s, 1H), 7.05 (s, 1H), 6.32 (d, J = 9 Hz, 1H), 6.03 (s, 1H), 4.52 - 4.48 (m, 1H), 4.37 - 4.35 (m, 4H), 3.94 (d, J = 8.5 Hz, 1H), 3.55 (t, J = 7 Hz, 1H), 3.23 (q, J = 7.2 Hz, 1H), 2.80-2.65 (m, 3H), 2.14 - 2.12 (m, 3H), 1.96 -1.94 (m, 3H), 1.79-1.71 (m, 3H),1.15(d,J=7 Hz,3H) 0.74 (d, J = 6.5Hz, 3H).

**Example 7 Synthesis of (2S)-1-(2-(3-ethyl-5-isopropyl-2, 4-dioxoimidazolidin-1-yl)-5, 6-dihydrobenzo[f] imidazo [1, 2-d] [1, 4] oxazepin-9-yl) pyrrolidine-2-carboxamide (compound 7)**

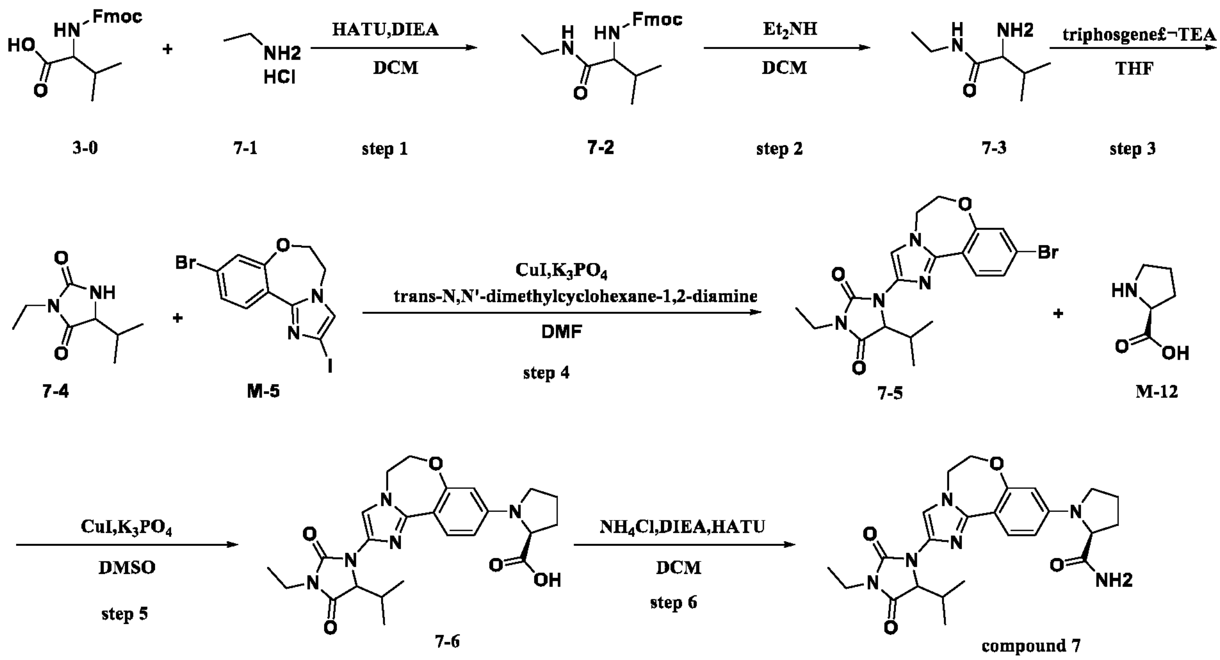


Replacing the compound 3-1 with compound 7-1, compound 7 was prepared following similar procedures as described in Example 3.

LC-MS $[M+H]^+$: 467.

The compounds in Table 1 were prepared in accordance with the synthetic protocols set forth in Example 1-7,

using the appropriate starting materials and suitable reagents.

Table 1

| EX. NO. | Structure | Chemical Name | LCMS $[M+H]^+$ |
|---|---|---|---|
| 8 | 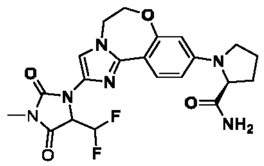 | (2S)-1-(2 -(5-difluoromethyl-3-methyl-2,4-dioxoimidazolidin-1-yl)-5,6-dihydrobenzo[f]imidazo[1,2-d] [1,4] oxazepin-9-yl)pyrrolidine-2-carboxamide | 461 |
| 9 | 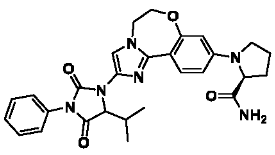 | (2S)-1-(2-(5-isopropyl-2,4-dioxo-3-phenylimidazolidine-1-yl)-5,6-dihydrobenzo[f]imidazo[1,2-d] [1,4] oxazepin-9-yl) pyrrolidine-2-carboxamide | 515 |
| 10 | 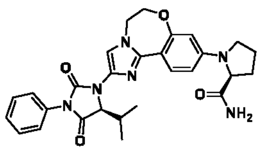 | (S)-1-(2-((S)-5-isopropyl-2,4-dioxo-3-phenylimidazolidine-1-yl)-5,6-dihydrobenzo[f]imidazo[1,2-d] [1,4]oxazepin-9-yl) pyrrolidine-2-carboxamide | 515 |
| 11 | 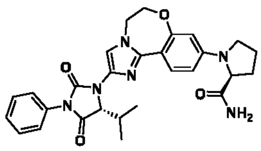 | (S)-1-(2-((R)-5-isopropyl-2,4-dioxo-3-phenylimidazolidine-1-yl)-5,6-dihydrobenzo[f]imidazo[1,2-d] [1,4]oxazepin-9-yl) pyrrolidine-2-carboxamide | 515 |
| 12 | 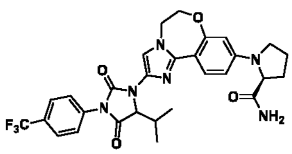 | (2S)-1-(2-(5-isopropyl-2,4-dioxo-3-(4-(trifluoromethyl) phenyl)imidazolidine-1-yl)-5,6-dihydrobenzo[f]imidazo[1,2 -d] [1,4] oxazepin-9-yl)pyrrolidine-2-carboxamide | 583 |
| 13 | 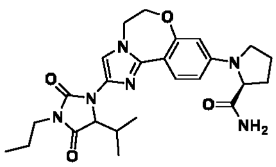 | (2S)-1-(2-(5-isopropyl-2,4-dioxo-3-propylimidazolidine-1-yl)-5,6-dihydrobenzo[f]imidazo[1,2-d] [1,4] oxazepin-9-yl) pyrrolidine-2-carboxamide | 481 |
| 14 | 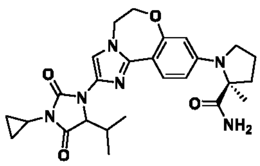 | (2S)-1-(2-(3-cyclopropyl -5-isopropyl)-2,4-dioxoimidazolidin-1-yl)-5,6-dihydrobenzo[f]imidazo[1,2-d] [1,4] oxazepin-9-yl)-2-methylpyrrolidine-2-carboxamide | 493 |
| 15 | 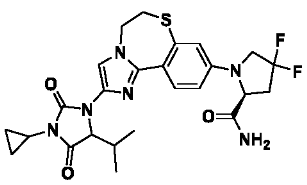 | (2S)-1-(2-(3-cyclopropyl -5-isopropyl)-2,4-dioxoimidazolidin-1-yl)-3,6-dihydrobenzo[f]imidazo[1,2-d] [1,4] thiazepin-9-yl)-4,4-difluoropyrrolidine-2-carboxamide | 531 |

(continued)

| EX. NO. | Structure | Chemical Name | LCMS [M+H]+ |
|---|---|---|---|
| 16 | 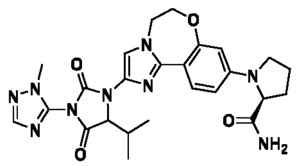 | (2S)-1-(2-(5-isopropyl-3-(1-methyl-1H-1,2,4-triazol-5-yl)-2,4-dioxoimidazolidin-1-yl)-5,6 -dihydrobenzo[f]imidazo [1,2-d] [1,4]oxazepin-9-yl)pyrrolidine-2-carboxamide | 520 |
| 17 | 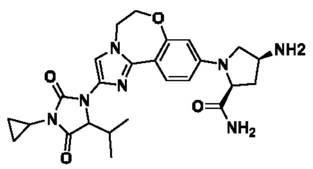 | (2S,4S)-4-amino-1-(2-(3-cyclopropyl -5-isopropyl-2,4-dioxoimidazolidin-1-yl)-5,6-dihydrobenzo[f] imidazo [1,2-d ] [1,4]oxazepin-9-yl)pyrrolidine-2-carboxamide | 494 |
| 18 | 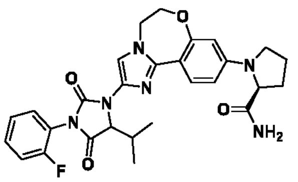 | (2S)-1-(2-(3-(2-fluorophenyl)-5-isopropyl-2,4-dioxoimidazolidin-1-yl)-5,6-dihydrobenzo[f]imidazo[1,2-d] [ 1,4]oxazepin-9-yl)pyrrolidine-2-carboxamide | 533 |
| 19 | 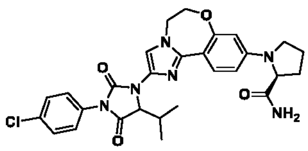 | (2S)-1-(2-(3-(4-chlorophenyl)-5-isopropyl-2,4-dioxoimidazolidin-1-yl)-5,6-dihydrobenzo[f]imidazo[1,2-d] [ 1,4] oxazepin-9-yl)pyrrolidine-2-carboxamide | 549 |
| 20 | 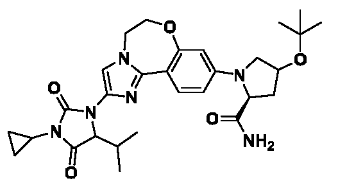 | (2S)-4-(tertbutoxy)-1-(2-(3-cyclopropyl - 5-isopropyl-2,4-dioxoimidazolidin-1-yl)-5,6-dihydrobenzo[f] imidazo [1,2] -d] [1,4]oxazepin-9-yl)pyrrolidine-2-carboxamide | 551 |
| 21 | 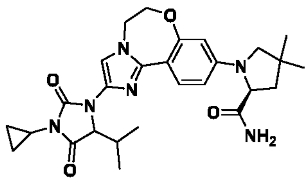 | (2S)-1-(2-(3-cyclopropyl-5-isopropyl)-2,4-dioxoimidazolidin-1-yl)-5,6-dihydrobenzo[f]imidazo[1,2-d] [1,4]oxazepin-9-yl)-4,4-dimethylpyrrolidine-2-carboxamide | 507 |
| 22 | 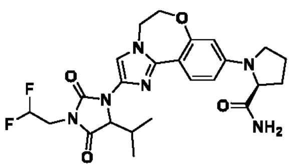 | (2S)-1-(2-(3-(2,2-difluoroethyl)-5-isopropyl-2,4-dioxoimidazolidin-1-yl)-5,6-dihydrobenzo[f]imidazo[1,2-d ] [1,4]oxazepin-9-yl)pyrrolidine-2-carboxamide | 503 |
| 23 | 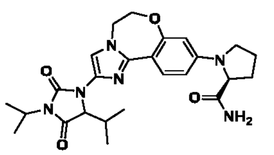 | (2S)-1-(2-(3,5-diisopropyl-2,4-dioxoimidazolidin-1-yl)-5,6-dihydrobenzo[f]imidazo[1,2-d] [1,4] oxazepin- 9-yl) pyrrolidine-2-carboxamide | 481 |

(continued)

| EX. NO. | Structure | Chemical Name | LCMS [M+H]+ |
|---|---|---|---|
| 24 | 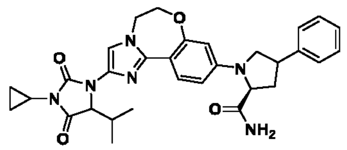 | (2S)-1-(2-(3 -cyclopropyl-5 -isopropyl)-2,4-dioxoimidazolidin-1-yl)-5,6-dihydrobenzo[f]imidazo[1,2-d][1,4]oxazepin-9-yl)-4-phenylpyrrolidine-2-carboxamide | 555 |
| 25 | 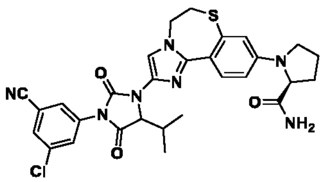 | (2S)-1-(2-(3-(3-chloro5-cyanophenyl)-5-isopropyl-2,4-dioxoimidazolidin-1-yl)-5,6-dihydrobenzo[f]imidazo[1,2 -d][1,4] thiazepin-9-yl)pyrrolidine-2-carboxamide | 590 |
| 26 | 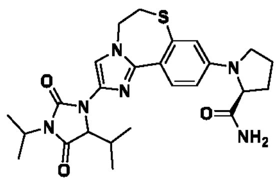 | (2S)-1-(2-(3,5-diisopropyl-2,4-dioxoimidazolidin-1-yl)-5,6-dihydrobenzo[f]imidazo[1,2-d] [1,4]thiazepin- 9-yl) pyrrolidine-2-carboxamide | 497 |
| 27 | 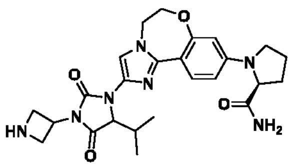 | (2S)-1-(2- (3-azetidin-3-yl)-5-isopropyl-2,4-dioxoimidazolidin-1-yl)-5,6-dihydrobenzo[f]imidazo[1,2-d][1,4] oxazepin-9-yl)pyrrolidine-2-carboxamide | 494 |
| 28 | 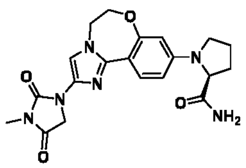 | (2S)-1-(2-(3-methyl-2,4-dioxoimidazolidin-1-yl)-5,6-dihydrobenzo[f]imidazo[1,2-d] [1,4] oxazepin-9-yl) pyrrolidine-2-carboxamide | 411 |
| 29 | 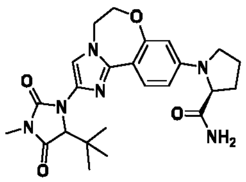 | (2S)-1-(2- (5-tertbutyl-3 -methyl-2,4-dioxoimidazolidin-1-yl)-5,6-dihydrobenzo[f]imidazo[1,2-d] [1,4] oxazepin-9-yl) pyrrolidine-2-carboxamide | 467 |
| 30 | 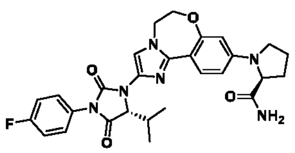 | (S)-1-(2-((R)-3-(4-fluorophenyl)-5-isopropyl-2,4-dioxoimidazolidin-1-yl)-5,6-dihydrobenzo[f]imidazo[1,2]-d][1,4]oxazepin-9-yl)pyrrolidine-2-carboxamide | 533 |
| 31 | 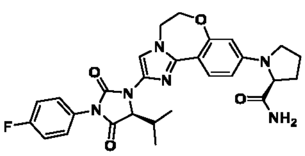 | (S)-1-(2-((S)-3-(4-fluorophenyl)-5-isopropyl-2,4-dioxoimidazolidin-1-yl)-5,6-dihydrobenzo[f]imidazo[1,2] -d][1,4]oxazepin-9-yl)pyrrolidine-2-carboxamide | 533 |

(continued)

| EX. NO. | Structure | Chemical Name | LCMS $[M+H]^+$ |
|---|---|---|---|
| 32 | 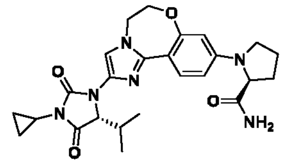 | (2S)-1-(2-((SR)-3-cyclopropyl -5-isopropyl-2,4-dioxoimidazolidin-1-yl)-5,6-dihydrobenzo[f]imidazo[1,2-d][1,4]oxazepin-9-yl)pyrrolidine-2-carboxamide | 479 |
| 33 | 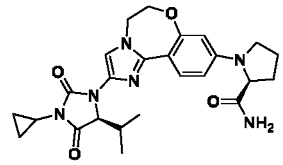 | (2S)-1-(2-((5S)-3-cyclopropyl -5-isopropyl-2,4-dioxoimidazolidin-1-yl)-5,6-dihydrobenzo[f]imidazo[1,2-d][1,4]oxazepin-9-yl)pyrrolidine-2-carboxamide | 479 |
| 34 | 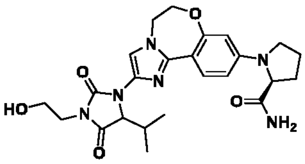 | (2S)-1-(2-(3-(2-hydroxyethyl)-5-isopropyl-2,4-dioxoimidazolidin-1-yl)-5,6-dihydrobenzo[f]imidazo[1,2-d][1,4]oxazepin-9-yl)pyrrolidine-2-carboxamide | 483 |
| 35 | 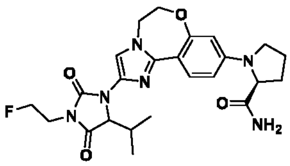 | (2S)-1-(2-(3-(2-fluoroethyl)-5-isopropyl-2,4-dioxoimidazolidin-1-yl)-5,6-dihydrobenzo[f]imidazo[1,2-d][1,4]oxazepin-9-yl)pyrrolidine-2-carboxamide | 485 |
| 36 | 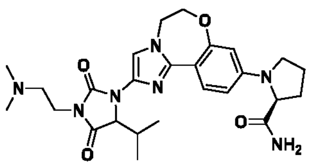 | (2S)-1-(2-(3-(2-(dimethylamino)ethyl)-5-)isopropyl-2,4-dioxoimidazolidin-1-yl)-5,6-dihydrobenzo[f]imidazo[1,2- d][1,4]oxazepin-9-yl)pyrrolidine-2-carboxamide | 510 |
| 37 | 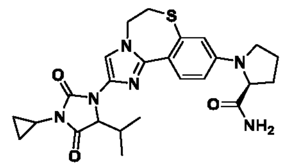 | (2S)-1-(2-(3-cyclopropyl -5-isopropyl)-2,4-dioxoimidazolidin-1-yl)-5,6-dihydrobenzo[f]imidazo[1,2-d][1,4]thiazepin-9-yl)pyrrolidine-2-carboxamide | 495 |
| 38 | 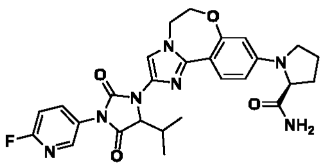 | (2S)-1-(2-(3-(6-fluoropyridin-3-yl)-5-isopropyl-2,4-dioxoimidazolidin-1-yl)-5,6-dihydrobenzo[f]imidazo[1,2 -d][1,4]oxazepin-9-yl)pyrrolidine-2-carboxamide | 534 |
| 39 | 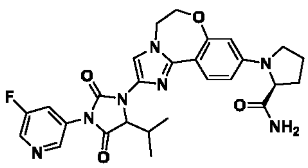 | (2S)-1-(2-(3-(5-fluoropyridin-3-yl)-5-isopropyl-2,4-dioxoimidazolidin1-yl)-5,6-dihydrobenzo[f]imidazo[1,2] -d][1,4]oxazepin-9-yl)pyrrolidine-2-carboxamide | 534 |

(continued)

| EX. NO. | Structure | Chemical Name | LCMS [M+H]+ |
|---|---|---|---|
| 40 | 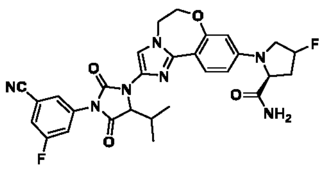 | (2S)-1-(2-(3-(3-cyano-5-fluorophenyl)-5-isopropyl-2,4-dioxoimidazolidin-1-yl)-5,6-dihydrobenzo[f]imidazo[1,2 -d][1,4]oxazepin-9-yl)-4-fluoropyrrolidine-2-carboxamide | 576 |
| 41 | 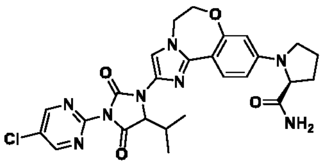 | (2S)-1-(2-(3-(3-chloropyrimidin-2-yl)-5-isopropyl-2,4-dioxoimidazolidin-1-yl)-5,6-dihydrobenzo[f]imidazo[1,2 -d][1,4]oxazepin-9-yl)pyrrolidine-2-carboxamide | 551 |
| 42 | 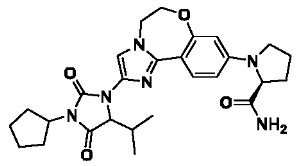 | (2S)-1-(2-(3-cyclopentyl-5-isopropyl-2,4-dioxoimidazolidin-1-yl)-5,6-dihydrobenzo[f]imidazo[1,2-d] [1,4] oxazepin-9-yl) pyrrolidine-2-carboxamide | 507 |
| 43 | 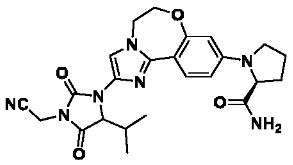 | (2S)-1-(2-(3-(cyanomethyl)-5-isopropyl-2,4-dioxoimidazolidin-1-yl)-5,6-dihydrobenzo[f]imidazo[1,2-d][1,4]oxazepin-9-yl)pyrrolidine-2-carboxamide | 478 |
| 44 | 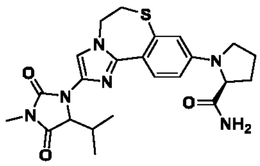 | (2S)-1-(2-(5-isopropyl-3-methyl-2,4-dioxoimidazolidin-1-yl)-5,6-dihydrobenzo[f]imidazo[1,2-d] [1,4] thiazepin-9-yl) pyrrolidine-2-carboxamide | 469 |
| 45 | 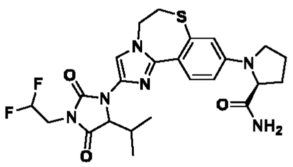 | (2S)-1-(2-(3-(2,2-difluoroethyl)-5-isopropyl-2,4-dioxoimidazolidin-1-yl)-5,6-dihydrobenzo[f]imidazo[1,2-d][1,4]thiazepin-9-yl)pyrrolidine-2-carboxamide | 519 |
| 46 | 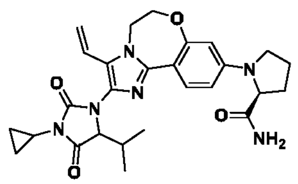 | (2S)-1-(2-(3-cyclopropyl -5-isopropyl-2,4-dioxoimidazolidin-1-yl)-3-vinyl-5,6-dihydrobenzo[f]imidazo[1,2-d] [ 1,4]oxazepin-9-yl)pyrrolidine-2-carboxamide | 505 |
| 47 | 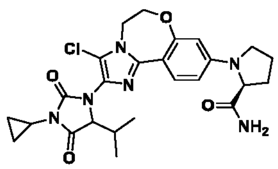 | (2S)-1-(3-chloro-2-(3-cyclopropyl -5-isopropyl-2,4-dioxoimidazolidin-1-yl)-5,6-dihydrobenzo[f]imidazo[1,2-d][1,4]oxazepin-9-yl)pyrrolidine-2-carboxamide | 513 |

(continued)

| EX. NO. | Structure | Chemical Name | LCMS [M+H]+ |
|---|---|---|---|
| 48 | 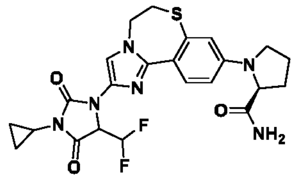 | (2S)-1-(2-(3-cyclopropyl -5-(difluoromethyl)-2,4-dioxoimidazolidin-1-yl)-5,6-dihydrobenzo[f]imidazo[1,2-d][1, 4]thiazepin-9-yl)pyrrolidine-2-carboxamide | 503 |
| 49 | 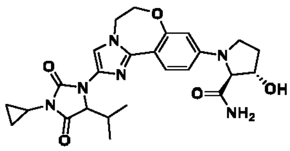 | (2S,3S)-1-(2-(3-cyclopropyl -5-isopropyl-2,4-dioxoimidazolidin-1-yl)-5,6-dihydrobenzo[f]imidazo[1,2-d][1,4]oxazepin-9-yl)-3 -hydroxy pyrrolidine-2-carboxamide | 495 |
| 50 | 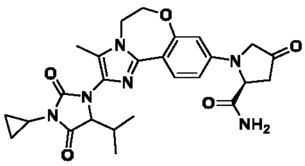 | (2S)-1-(2-(3-cyclopropyl -5-isopropyl-2,4-dioxoimidazolidin-1-yl)-3 -methyl-5,6-dihydrobenzo[f]imidazo[1,2-d] [1,4]oxazepin-9-yl)-4-oxopyrrolidine-2-carboxamide | 507 |
| 51 | 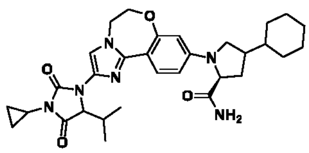 | (2S)-4-cyclohexyl-1-(2-(3-cyclopropyl-5-isopropyl-2,4-dioxoimidazolidin-1-yl)-5,6-dihydrobenzo[f]imidazo[1,2-d][1,4]oxazepin-9-yl)pyrrolidine-2-carboxamide | 561 |
| 52 | 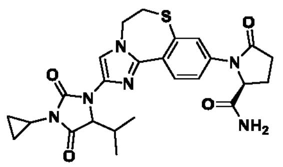 | (2S)-1-(2-(3-cyclopropyl -5-isopropyl-2,4-dioxoimidazolidin-1-yl)-5,6-dihydrobenzo[f]imidazo[1,2-d][1,4] thiazepin-9-yl)-5-oxopyrrolidine-2-carboxamide | 509 |

All the racemic compounds in Table 1 can be synthesized using the corresponding chiral raw materials to obtain the corresponding enantiomers, or the enantiomers were separated on a chiral column basically according to the method described in Step7 in Example 2.

**Control Compound**

Table 2

| EX. | Compound name | Structure |
|---|---|---|
| 1 | (2S)-1-(2 -((4R)-4-methyl-2-oxooxazolidin -3-yl)-5,6-dihydrobenzo[f]imidazo [1,2-d] [1,4] oxazepin -9-yl)pyrrolidine-2-carboxamide | 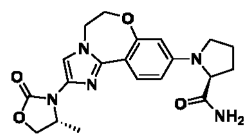 |
| 2 | (2S)-1-(2-((2R)-2-(hydroxymethyl)-5-oxopyrrolidine-1-yl)-5,6-dihydrobenzo[f] imidazo[1,2-d] [1,4] oxazepin-9-yl)pyrrolidine-2-carboxamide | 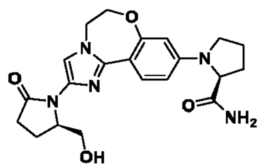 |

(continued)

| EX. | Compound name | Structure |
|---|---|---|
| 3 | (2S)-1-(2-(2-(1,1,1-trifluoro-2-methylpropan-2-yl)pyridn-4-yl)-5,6-dihydrobenzo [f]imidazo[1,2-d] [1,4] oxazepin-9-yl)pyrrolidine-2-carboxamide | 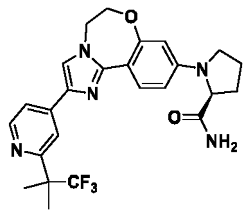 |

Control compound 1 (Example 102 in WO2017001658 ) and control compound 2 were prepared in accordance with the synthetic protocols set forth in WO2017001658, using the appropriate starting materials and intermediate and agents; The method to prepare the control compound 3 are as follows.

Preparation of control compound 3

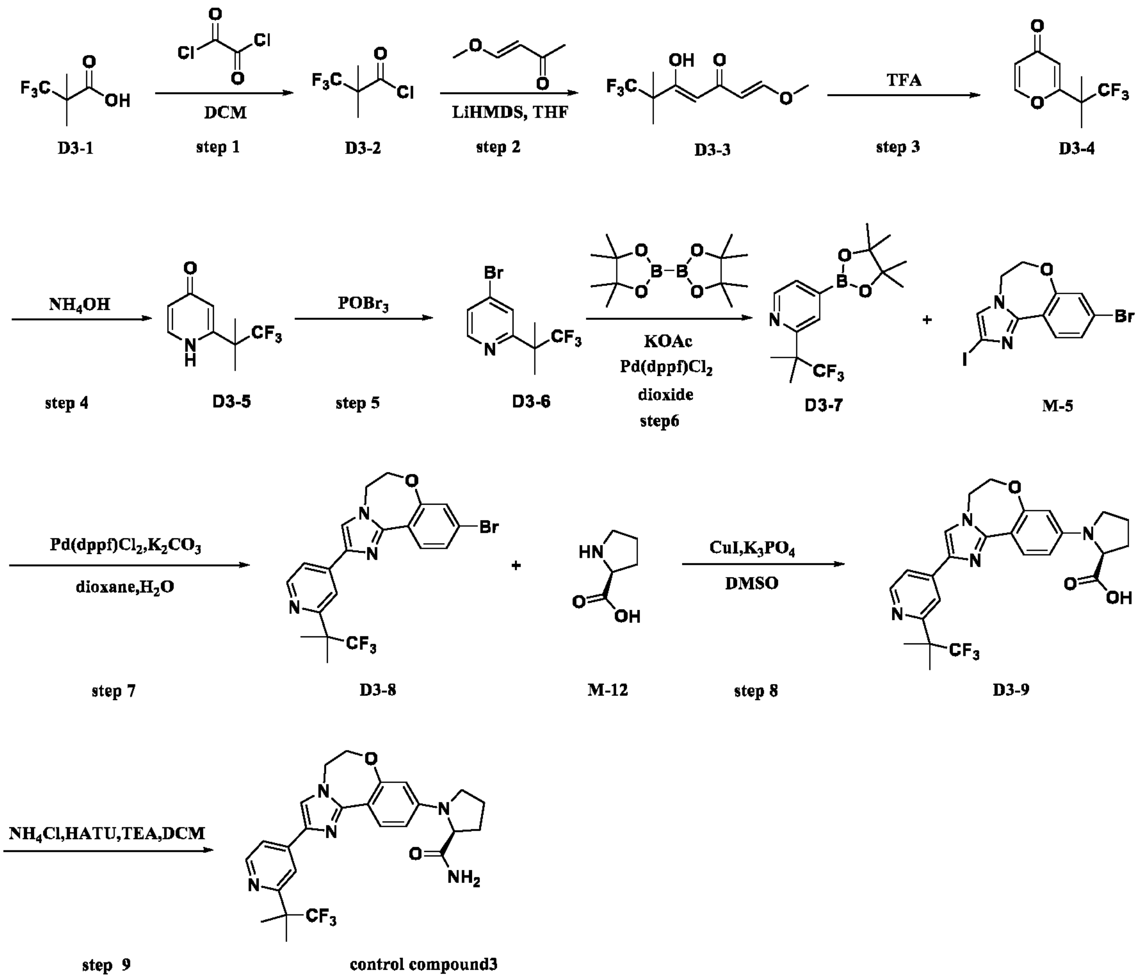


**Step 1: Synthesis of compound D3-2**

Compound D3-1 (2000 g) and oxalyl chloride (2019g) were dissolved in dichloromethane (8 L). DMF (5mL) was added to the reaction slowly, refluxed for 6 hours. The reaction system was concentrated to give 2245 g suspension containing yellow solid, which was used in the next step directly.

**Step 2: Synthesis of compound D3-3**

To a solution of 4-methoxy-3-buten-2-one (642 g) in anhydrous THF(16 L), 6.42 L LiHMDS(1 mol/L) dissolved in THF was added dropwise at -78°C. After stirred at -78°C for 2 h, suspension in step 1 dissolved in THF(1 L) was added to the reaction system slowly, stirred at -78°C for 1-2 h, the reaction mixture was heated to room temperature and stirred for 16 h. The resulting reaction mixture was used in the next step directly.
LC-MS $[M+H]^+$ : 239.

**Step 3: Synthesis of compound D3-4**

The reaction mixture in Step 2 was quenched with the addition of water (500 mL) in an ice bath, and then trifluoroacetic acid (50mL) was added, stirred at room temperature for 3h. When the reaction was completed, the reaction mixture was concentrated and extracted with EA.The organic layers were washed with brine, dried over anhydrous $Na_2SO_4$ and concentrated. The residue was purified by column chromatography (Hex/EA 100:1-10:1) to give brown solid. The brown solid was washed with a mixed solvent of HEX and EA (Volume ratio 50:1), the reaction mixture was filtered off with suction, the filtrate was collected and concentrated, to give compound D3-4 (395g).
LC-MS $[M+H]^+$: 207.
$^1$H NMR (MeOD-d6): 1.59 (s, 6H), 6.39-6.41 (m, 1H), 6.53-6.54 (m, 1H), 8.12-8.14 (m, 1H).

**Step 4: Synthesis of compound D3-5**

Compound D3-4(395 g) was added to 2 L of ammonia (30%), stirred and refluxed for 2 h. The reaction mixture was cooled, concentrated, to give 343.00 g compound D3-5, which was used in the next step directly.
LC-MS $[M+H]^+$: 206.

**Step 5: Synthesis of compound D3-6**

The mixture of compound D3-5 (343 g) and phosphorus oxybromide (966.68 g) was heated to 120 °C and stirred for 1 h. The reaction mixture was poured into the ice water whilst hot. Sodium bicarbonate was added to adjust pH to neutral, the reaction mixture was extracted with EA. The organic layers were dried and concentrated. The residue was purified by column chromatography (Hex/EA=100:1) to give compound D3-6 (305 g).
LC-MS $[M+H]^+$: 269.
$^1$H NMR (MeOD-d6): 1.59 (s, 6H), 7.52-7.54 (m, 1H), 7.78 (s, 1H), 8.41-8.42(m, 1H).

**Step 6: Synthesis of compound D3-7**

Bis(Pinacolato)diborane (710.45 mg), compound D3-6 (500 mg), potassium acetate (1.88 g), $[PdCl_2(dppf)]CH_2Cl_2$(152.32 mg) and dioxane (20 mL) were added to a single-neck flask under nitrogen protection, reacted in a 90 °C oil bath for 4h. The reaction mixture was concentrated. The residue was purified by column chromatography (Hex/EA=3:1) to give compound D3-7 (450 mg).
LC-MS $[M+H]^+$: 316.

**Step 7: Synthesis of compound D3-8**

$[PdCl_2(dppf)]CH_2Cl_2$ (233.22 mg), potassium carbonate (394.70 mg), compound D3-7 (450 mg), compound M-5 (586.25 mg), dixone (3 mL) and $H_2O$ (0.5 mL) were added to a single-neck flask under nitrogen protection, refluxed in a 70 °C oil bath for 15 h. The reaction mixture was concentrated.The residue was purified by column chromatography (PE: EA=50:50) to give compound D3-8 (130mg).
LC-MS $[M+H]^+$: 452.

**Step 8: Synthesis of compound D3-9**

The compound D3-9 was prepared in accordance with the synthetic protocols set forth in step 5 in example 2.
LC-MS $[M+H]^+$: 487.

**Step 9: Synthesis of control compound 3**

Control compound 3 was prepared in accordance with the synthetic protocols set forth in step 6 in example 2.

LC-MS [M+H] +: 486.

**Pharmacological experiment**

The following experiments show that the preferred compounds of the present invention inhibits kinase activities of PI3Kα effectively *in vitro.* The anti-proliferative activities against tumor cells haoboring PI3Kα mutants and the pharmacokinetic profile of the preferred compound in the present invention are better than the control compound with significant superiority.

**Example A: Kinase experiment**

PI3Kα, PI3Kβ, PI3Kγ kinases undergo enzymatic reactions with their substrates ATPand PIP2:3PS, the amout of the product detected by ADP-Glo agent and luminescence method were used to reflect the enzymatic activities of PI3Kα, PI3Kβ, PI3Kγ (the final concentration of ATP is 10 μM).The above methods were used to test the inhibitory activity of certain compound of the present invention on PI3Kα, PI3Kβ and PI3Kγ kinase.
Method:

Agent: Basic kinase buffer (pH 7.5); PI3Kα, PI3Kβ, PI3Kγ kinase solutions; PIP2:3PS and ATP solutions; ADP-Glo kit (containing10 mM $MgCl_2$).
Wherein, the buffer composition: 50 mM Hepes (pH7.2- 7.5), 3 mM $MgCl_2$, 1 mM EGTA, 0.03% CHAPS, 100 mM NaCl, 2 mM DTT;
Compound preparation: The tested compound was diluted to a specific concentration with 100% DMSO.

Process:

1) PI3Kα, PI3Kβ, or PI3Kγ protein solution were dispensed to a 384-well assay plate (6008280, PerkinElmer), and centrifuged at 1000 rpm for 1 minute.
2)Tested compound, negative control DMSO or positive control BYL719 was dispensed to the above-metioned 384-well assay plate with the kinase,centrifuged at 1000 rpm for 1 min, and incubated at 25 °C for 15 mins.
3) PIP2:3PS&ATP solution was added to the above-mentioned 384-well assay plate, centrifuged at 1000 rpm for 1 min, incubated at 25 °C for 60 mins.
4) 5 μL of ADP-Glo agent (10 mM $MgCl_2$ contained) was transferred to the 384-well assay plate, centrifuged at 1000 rpm for 1 min, incubated at 25 °C for 40mins.
5) 10 μL of Detection agent was transferred to the 384-well assay plate, centrifuged at 1000 rpm for 1 min, incubated at 25 °C for 40mins.
6) RLU (Relative luminescence unit) value was measured by Envision multifunctional plate reader. The RLU value was used to characterize the degree of reaction between the enzyme and the substrate, and calculate the $IC_{50}$ value.
7) Data analysis:

Compound inhibition rate (% inh) = (negative control RLU-tested compound RLU)/(negative control RLU-positive control RLU)*100%

The $IC_{50}$ (half inhibitory concentration) of the compound was obtained using the following nonlinear fitting formula:

Y=minimum inhibition rate + (max inhibition rate-minimum inhibition rate)/(1+10^((Log$IC_{50}$-X)* slope)); wherein, X is the log value of the concentration of the tested compound, Y is the inhibition rate of tested compound (% inh).

The results of some examplesof the present invention are shown in Table 3.Wherein, A represents the $IC_{50}$ value≤5nM; B represents the $IC_{50}$ value is 5~20nM; C represents the $IC_{50}$ value is 300~600nM; D represents the $IC_{50}$ value > 600nM.

Table 3

| Example | $IC_{50}$ of compound to PI3K (nM) | | |
|---|---|---|---|
| | α | β | γ |
| Example 1 | A | D | D |
| Example 2 | A | D | C |
| Example 3 | A | / | / |
| Example 4 | B | D | D |
| Example 5 | B | / | / |
| Example 6 | B | / | / |
| Example 7 | B | / | / |
| Example 9 | B | / | / |
| Example 23 | A | / | / |
| Example 32 | A | C | C |
| Example 33 | A | D | D |
| Note: "/"represents not tested. | | | |

As Table 3 shows, the compound provided by the present invention have favorable PI3Kα kinase inhibitory activities, and good selectivity from PI3Kβ and PI3Kγisoforms, which can avoid the potential adverse effects caused by multi-target inhibition.

**Example B: Cell proliferation Assay**

Method: The anti-proliferative effects on human tumor cells MCF-7 and HGC27 of certain compounds of the present invention were evaluated by CellTiter Glo assay.

Detection method: MCF-7 cells were suspended in DMEM culture medium, the cell concentration of which was adjusted to 25000 cells/mL; HGC27 cells were suspended in RPMI-1640 culture medium, the cell concentration of which was adjusted to 5000cells/mL. 100 μL of cell suspension was dispensed to a 96-well assay plate and placed in a $CO_2$ incubator overnight.The tested compounds were dissolved in DMSO, and 3-fold derail dilution was performed to obtain 10-point concentrations. the 10-point concentrations of the tested compounds or or negative control was transferred to the well containing 100 μL of culture medium respectively, incubated at 37 °C, 5% $CO_2$, the HGC27 cells were incubated for 96h, MCF-7 cells were incubated for 120h. Then 100 μL of CellTiter-Glo agent was added to the above-mentioned 96-well assay plate, incubated at room temperature for 10 minutes to allow stable luminescence signal. VICTOR TM X5 instrument was used to record RLU (relative luminescence unit), and then calculate the $IC_{50}$ values were calculated.

Compound inhibition rate (% inh) =100% - (tested compound RLU-blank control RLU)/ (negative control RLU-blank control RLU)*100%

Negative control: DMSO;

Blank control: blank culture medium, with no compound or cell;

The $IC_{50}$ (half inhibitory concentration) of the compound was obtained using the following nonlinear fitting formula:

Y=minimum inhibition rate + (max inhibition rate-minimum inhibition rate)/(1+10^((Log$IC_{50}$-X)*slope)); wherein, X: the log value of the concentration of the tested compound ; Y : the inhibition rate of the tested compound (% inh).

Experiment data are shown in Table 4 andTable 5.

Table 4 $IC_{50}$ value of Example compounds on MCF cell

| Example | $IC_{50}$(μM) of compound on MCF cell |
|---|---|
| Control compound 1 | 0.444 |
| Control compound 2 | 0.549 |
| Control compound 3 | 0.519 |
| Example 1 | 0.176 |
| Example 2 | 0.070 |
| Example 5 | 0.373 |
| Example 9 | 0.142 |
| Example 23 | 0.074 |
| Example 28 | 0.468 |
| Example 32 | 0.107 |
| Example 33 | 0.047 |

Table 5 $IC_{50}$ value of Example compounds on HGC-27 cell

| Example | $IC_{50}$(μM) of ompound on HGC-27cell |
|---|---|
| Control compound 1 | 1.579 |
| Control compound 3 | 2.067 |
| Example 2 | 0.361 |
| Example 5 | 4.071 |
| Example 28 | 3.921 |
| Example 32 | 0.415 |
| Example 33 | 0.267 |

From the above table, the compounds provided by the present invention have a better cell proliferation inhibitory activities for cell lines carring PI3Kα point mutations than the control compounds, and have better anti-tumor effects at the same concentration. Therefore, the compound provided by the present invention is expected to be a PI3Kαkinase inhibitor with better anti-tumor effects.

**Example C: Pharmacokinetic test**

Method: 42 male SD rats, weight: 150-300g.Divided into 7 groups randomly, with 6 rats in each group. Among the 6 rats in each group, three of them were administrated a single intravenous injection of 2 mg/mL of the example compound, the other three were given intragastric administration of 10mg/mL of the example compound, blood was collected from the orbital venous plexus at designated time points, plasma was separated, and stored in a -80 °C refrigerator for later use.

The protein of the resultant plasma was precipitated by acetonitrile, the supernatant was extracted, and mixed with water at the ratio of 1:1, took 10μL of which for LC-MS/MS detection, and calculated the average was calculated. The results are shown in Table 6.

Table 6 Pharmacokinetic Test Results of Example Compounds

| Compound No. | Intravenous injection | | Gavage | | |
|---|---|---|---|---|---|
| | Dosage (mg/kg) | CL (mL/min/kg) | Dosage (mg/kg) | Cmax (ng/mL) | AUC (hr*ng/mL) |
| Control compound1 | 2 | 16.6 | 10 | 1963 | 7882 |
| Example 2 | 2 | 1.98 | 10 | 4340 | 52993 |
| Example 4 | 2 | 4.75 | 10 | 2690 | 29080 |
| Example 23 | 2 | 8.76 | 10 | 1833 | 18563 |
| Example 32 | 2 | 1.5 | 10 | 5033 | 66414 |
| Example 33 | 2 | 11.1 | 10 | 1597 | 19832 |

According to relevant statistics (Kola I, Landis J. Nat Rev Drug Discov. 2004 Aug;3(8):711-5.), about 40% of drug candidates failed clinical development due to poor PK/bioavailability in the early 1990s, therefore PK/bioavailability plays a very critical role in the clinical development of candidate drugs.

The above table shows that compounds provided by the present invention have unexpectedly higher oral exposure and lower in vivo clearance compared to the control compounds, which leads to higher systemic exposure at the same dose. The compound of the present invention achieves the same anti-tumor effects at a lower dose level, thereby reduces the probability of toxicity and safety risks, and increase the druggability of the compound as well.

**Claims**

**1.** A compound of Formula (I) or a stereoisomer, geometric isomer or tautomer, or pharmaceutically acceptable salt, solvate, chelate, non-covalent complex or prodrug thereof,

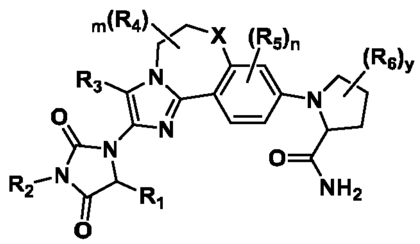

Formula (I)

wherein,

X is selected from O and S;

$R_1$ is selected from H, CN, $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl, $C_{5-8}$ heteroaryl, $OR_a$ and $-NR_aR_b$; wherein $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, $OR_a$ , oxo, $C_{1-6}$ alkyl, $C_{2-6}$ alkenyl, $C_{2-6}$ alkynyl, $C_{3-6}$ cycloalkyl and $C_{3-6}$ heterocyclyl;

$R_2$ is selected from H, $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl, $C_{5-8}$ heteroaryl; wherein $C_{1-6}$ alkyl, $C_{2-6}$ alkenyl, $C_{2-6}$ alkynyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, -OH, $-NO_2$, $C_{1-6}$ alkyl, $C_{1-6}$ haloalkyl, $C_{2-6}$ alkenyl, $C_{2-6}$ haloalkenyl, $C_{2-6}$ alkynyl, $C_{2-6}$ haloalkynyl, $C_{3-6}$ cycloalkyl, $C_{3-6}$ halocycloalkyl, $C_{3-6}$ heterocyclyl, $C_{3-6}$ haloheterocyclyl, $C_{6-8}$ aryl, $C_{6-8}$ haloaryl, $C_{5-8}$ heteroaryl, $C_{5-8}$ haloheteroaryl, oxo, $-OR_a$, - $NR_aR_b$, $-C(O)R_a$, -C(O)O $R_a$, $-C(O)NR_aR_b$, $-S(O)R_a$ and-$S(O)_2R_a$;

$R_3$ is selected from H, halogen, CN, $C_{1-6}$ alkyl, $C_{2-6}$ alkenyl, $C_{2-6}$ alkynyl, $-OR_a$ and-$NR_aR_b$; wherein $C_{1-6}$ alkyl, $C_{2-6}$ alkenyl, $C_{2-6}$ alkynyl are each optionally substituted with one or more substituents selected from halogen, CN, $-OR_a$, $-NR_aR_b$, $-C(O)R_a$, $-C(O)OR_a$, $-C(O)NR_aR_b$, - $S(O)R_a$ and $-S(O)_2R_a$;

$R_4$ is selected from H, halogen, CN, $C_{1-6}$ alkyl, $C_{2-6}$ alkenyl, $C_{2-6}$ alkynyl, oxo, $C_{1-6}$ haloalkyl, $C_{2-6}$ haloalkenyl, $C_{2-6}$ haloalkynyl, $C_{1-6}$ alkoxy, $C_{1-6}$ halo alkoxy, $-OR_a$, $-NR_aR_b$, $-C(O)R_a$, - $C(O)OR_a$, $-C(O)NR_aR_b$, $-S(O)R_a$ and $-S(O)_2R_a$;

$R_5$ is selected from H, halogen, CN, $C_{1-6}$ alkyl, $C_{2-6}$ alkenyl, $C_{2-6}$ alkynyl, $C_{1-6}$ haloalkyl, $C_{2-6}$ haloalkenyl, $C_{2-6}$ haloalkynyl, $C_{1-6}$ alkoxy, $C_{1-6}$ haloalkoxy, $-OR_a$, $-NR_aR_b$, $-S(O)R_a$ and $-S(O)_2R_a$;
$R_6$ is selected from H, halogen, CN, oxo, $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl, $C_{5-8}$ heteroaryl, $-OR_a$, $-NR_aR_b$, $-C(O)R_a$, $-C(O)O R_a$, $- C(O) NR_aR_b$, $-S(O)R_a$ and $-S(O)_2R_a$; wherein $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl and $C_{5-8}$ heteroaryl are each optionally substituted with one or more substituents selected from halogen, CN, oxo, $-NO_2$, $C_{1-6}$ alkyl, $C_{2-6}$ alkenyl, $C_{2-6}$ alkynyl, $-OR_a$, $-NR_aR_b$, $-C(O)R_a$, $-C(O)O R_a$, $-C(O)NR_aR_b$, $-S(O)R_a$ and $-S(O)_2R_a$; or, two $R_6$ taken with the C atoms which they are attached to form $C_{3-6}$ cycloalkyl or $C_{3-6}$ heterocyclyl;
$R_a$ and $R_b$ are independently selected from H, $C_{1-6}$alkyl, $C_{2-6}$alkenyl, $C_{2-6}$alkynyl, $C_{1-6}$ alkoxy, $C_{3-6}$cycloalkyl, $C_{3-6}$heterocyclyl, $C_{6-8}$aryl, $C_{5-8}$heteroaryl; wherein $C_{1-6}$ alkyl, $C_{2-6}$ alkenyl, $C_{2-6}$ alkynyl, $C_{1-6}$ alkoxy, $C_{3-6}$ cycloalkyl, $C_{3-6}$ heterocyclyl, $C_{6-8}$ aryl, $C_{5-8}$ heteroaryl are optionally substituted with halogen, CN, -OH, $-NH_2$, $C_{1-6}$ alkyl, $C_{1-6}$ alkoxy, $C_{1-6}$ haloalkyl, $C_{1-6}$ haloalkoxy;
m is selected from 0, 1, 2, 3 and 4;
n is selected from 0, 1, 2 and 3; and
y is selected from 0, 1, 2, 3, 4, 5 and 6.

**2.** The compound of calim 1, or a stereoisomer, geometric isomer or tautomer, or a pharmaceutically acceptable salt, solvate, chelate, noncovalent complex or prodrug thereof, wherein $R_1$ is $C_{1-6}$ alkyl or $C_{3-6}$cycloalkyl, said $C_{1-6}$ alkyl and $C_{3-6}$ cycloalkyl can be substituted optionally and independently with halogen.

**3.** The compound of any one of claims 1-2, or a stereoisomer, geometric isomer or tautomer, or a pharmaceutically acceptable salt, solvate, chelate, noncovalent complex or prodrug thereof, wherein $R_2$ is selected from H, $C_{1-6}$ alkyl, $C_{3-6}$ cycloalkyl, $C_{3-6}$ heterocyclyl, $C_{6-8}$ aryl and $C_{5-8}$ heteroaryl; said $C_{1-6}$ alkyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, - CN, -OH, $-NR_aR_b$ , $C_{1-6}$alkyl and $C_{1-6}$ haloalkyl; said $R_a$ and $R_b$ are independently selected from H and $C_{1-6}$ alkyl.

**4.** The compound of any one of claims 1-3, or a stereoisomer, geometric isomer or tautomer, or a pharmaceutically acceptable salt, solvate, chelate, noncovalent complex or prodrug thereof, wherein $R_3$ is selected from H, halogen, CN, $C_{1-6}$ alkyl, $C_{2-6}$ alkenyl, $C_{2-6}$ alkynyl, $C_{1-6}$ haloalkyl, $C_{2-6}$ haloalkenyl and $C_{2-6}$ haloalkynyl.

**5.** The compound of any one of claims 1-4, or a stereoisomer, geometric isomer or tautomer, or a pharmaceutically acceptable salt, solvate, chelate, noncovalent complex or prodrug thereof, wherein $R_6$ is selected from H, halogen, CN, $-NH_2$, oxo, -OH, $C_{1-6}$ alkyl, $C_{2-6}$ alkenyl, $C_{2-6}$ alkynyl, $C_{1-6}$ alkoxy, $C_{1-6}$ haloalkyl, $C_{2-6}$ haloalkenyl, $C_{2-6}$ haloalkynyl, $C_{3-6}$ cycloalkyl, $C_{3-6}$ heterocyclyl, $C_{6-8}$ aryl and $C_{5-8}$ heteroaryl.

**6.** The compound of any one of claims 1-5, or a stereoisomer, geometric isomer or tautomer, or a pharmaceutically acceptable salt, solvate, chelate, noncovalent complex or prodrug thereof, wherein the compound is of Formula (II):

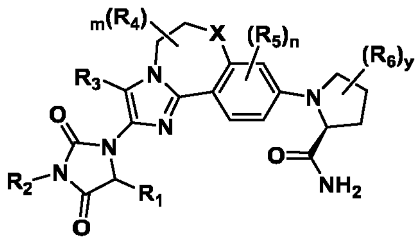

Formula (II).

**7.** The compound of any one of claims 1-6, or a stereoisomer, geometric isomer or tautomer, or a pharmaceutically acceptable salt, solvate, chelate, noncovalent complex or prodrug thereof, wherein the compound is of Formula(III-1):

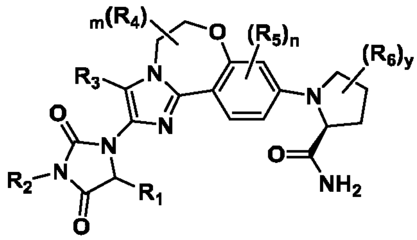

Formula (III-1)

wherein, $R_1$ is $C_{1-6}$ alkyl or $C_{1-6}$ haloalkyl.

8. The compound of any one of claims 1-6, or a stereoisomer, geometric isomer or tautomer, or a pharmaceutically acceptable salt, solvate, chelate, noncovalent complex or prodrug thereof, wherein the compound is of Formula(III-2):

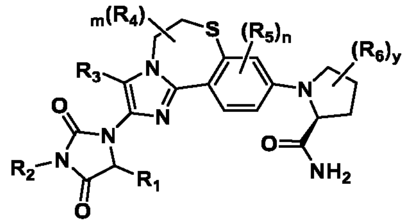

Formula (III-2).

9. The compound of any one of claims 1-8, or a stereoisomer, geometric isomer or tautomer, or a pharmaceutically acceptable salt, solvate, chelate, noncovalent complex or prodrug thereof, wherein $R_3$ is selected from H, halogen, $C_{1-6}$ alkyl and $C_{2-6}$ alkenyl.

10. The compound of any one of claims 1-9, or a stereoisomer, geometric isomer or tautomer, or a pharmaceutically acceptable salt, solvate, chelate, noncovalent complex or prodrug thereof, wherein $R_4$ and $R_5$ are both H.

11. The compound of any one of claims 1-6, or a stereoisomer, geometric isomer or tautomer, or a pharmaceutically acceptable salt, solvate, chelate, noncovalent complex or prodrug thereof, wherein the compound is of Formula(IV):

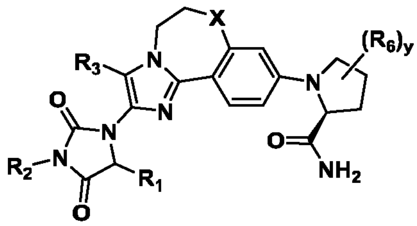

Formula (IV)

wherein:

$R_1$ is $C_{1-6}$alkyl or $C_{1-6}$ haloalkyl;
$R_2$ is selected from H, $C_{1-6}$ alkyl, $C_{3-6}$ cycloalkyl, $C_{3-6}$ heterocyclyl, $C_{6-8}$ aryl and $C_{5-8}$ heteroaryl; wherein $C_{1-6}$ alkyl, $C_{3-6}$ cycloalkyl, $C_{3-6}$ heterocyclyl, $C_{6-8}$ aryl and $C_{5-8}$ heteroaryl are each optionally substituted with one or more substituents selected from halogen, -CN, -OH, - $NR_aR_b$ , $C_{1-6}$alkyl, $C_{1-6}$haloalkyl, $C_{1-6}$ alkoxy and $C_{1-6}$ halo alkoxy;
$R_3$ is selected from H, halogen, $C_{1-6}$ alkyl and $C_{2-6}$ alkenyl;
$R_6$ is selected from H, halogen, $-NH_2$, oxo, -OH, $C_{1-6}$alkyl, $C_{1-6}$ alkoxy, $C_{1-6}$ haloalkyl, $R_{1-6}$ halo alkoxy, $C_{3-6}$ cycloalkyl and $C_{6-8}$ aryl;

$R_a$ and $R_b$ are independently selected from H and $C_{1-6}$ alkyl; and
y is selected from 0, 1, 2, 3, 4, 5 and 6.

**12.** The compound of claim 11, or a stereoisomer, geometric isomer or tautomer, or a pharmaceutically acceptable salt, solvate, chelate, noncovalent complex or prodrug thereof, wherein X is O.

**13.** The compound of any one of claims 1-7, or a stereoisomer, geometric isomer or tautomer, or a pharmaceutically acceptable salt, solvate, chelate, noncovalent complex or prodrug thereof, wherein the compound is of Formula(V):

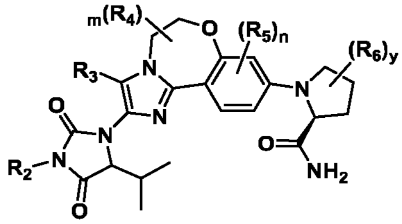

Formula (V).

**14.** The compound of any one of claims 1-13, or a stereoisomer, geometric isomer or tautomer, or a pharmaceutically acceptable salt, solvate, chelate, noncovalent complex or prodrug thereof, wherein $R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $C_{1-6}$ alkyl, $C_{3-6}$ cycloalkyl, $C_{3-6}$ heterocyclyl, $C_6$ aryl and $C_{5-6}$ heteroaryl are each optionally substituted with 1 or more substituents selected from halogen, - CN, -OH, $-N-(CH_3)_2$, $C_{1-6}$ alkyl and $C_{1-6}$ haloalkyl.

**15.** The compound of any one of claims 1-14, or a stereoisomer, geometric isomer or tautomer, or a pharmaceutically acceptable salt, solvate, chelate, noncovalent complex or prodrug thereof, wherein $R_6$ is selected from H, halogen, $-NH_2$, oxo, -OH, $C_{1-6}$ alkyl, $C_{1-6}$ alkoxy, $C_{3-6}$ cycloalkyl and $C_6$ aryl.

**16.** The compound of any one of claims 1-7, or a stereoisomer, geometric isomer or tautomer, or a pharmaceutically acceptable salt, solvate, chelate, noncovalent complex or prodrug thereof, wherein the compound is of Formula(VI):

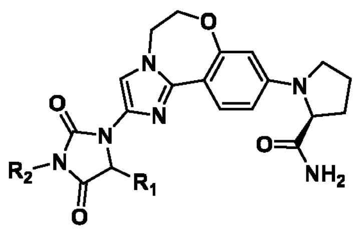

Formula (VI)

wherein,

$R_1$ is selected from $C_{1-6}$ alkyl and $C_{1-6}$ haloalkyl;
$R_2$ is selected from H, $C_{1-6}$ alkyl, $C_{3-6}$ cycloalkyl, $C_{3-6}$ heterocyclyl, $C_6$ aryl and $C_{5-6}$ heteroaryl; wherein $C_{1-6}$ alkyl, $C_{3-6}$ cycloalkyl, $C_{3-6}$ heterocyclyl, $C_6$ aryl and $C_{5-6}$ heteroaryl are each optionally substituted with one or more substituents selected from halogen, -CN, -OH, $-N-(CH_3)_2$, $C_{1-6}$ alkyl and $C_{1-6}$ haloalkyl.

**17.** The compound of any one of claims 1-16, or a stereoisomer, geometric isomer or tautomer, or a pharmaceutically acceptable salt, solvate, chelate, noncovalent complex or prodrug thereof, wherein $R_1$ is $C_{1-4}$ alkyl, wherein $R_1$ is substituted optionally with halogen.

**18.** The compound of any one of claims 1-17, or a stereoisomer, geometric isomer or tautomer, or a pharmaceutically acceptable salt, solvate, chelate, noncovalent complex or prodrug thereof, wherein $R_1$ is selected from$-CH(CH_3)_2$, $-C(CH_3)_3$, $-CF_3$ and $-CHF_2$.

**19.** The compound of any one of claims 1-18, or a stereoisomer, geometric isomer or tautomer, or a pharmaceutically acceptable salt, solvate, chelate, noncovalent complex or prodrug thereof, wherein $R_2$ is selected from H, $C_{1-6}$ alkyl, $C_{3-6}$ cycloalkyl, $C_{3-6}$ heterocyclyl, $C_6$ aryl and $C_5$-$C_6$ heteroaryl, wherein $R_2$ is substituted optionally with halogen.

**20.** The compound of any one of claims 1-19, or a stereoisomer, geometric isomer or tautomer, or a pharmaceutically acceptable salt, solvate, chelate, noncovalent complex or prodrug thereof, wherein $R_2$ is selected from $C_{1-6}$ alkyl, $C_{1-6}$ haloalkyl, $C_{3-6}$ cycloalkyl, $C_{3-6}$ halocycloalkyl, phenyl and halophenyl.

**21.** The compound of any one of claims 1-7, or a stereoisomer, geometric isomer or tautomer, or a pharmaceutically acceptable salt, solvate, chelate, noncovalent complex or prodrug thereof, wherein the compound is of Formula(VII):

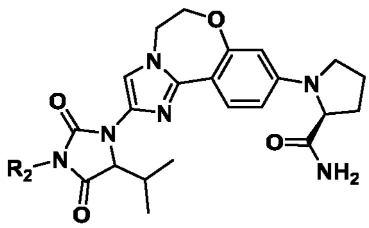

Formula (VII)

wherein, $R_2$ is selected from H, $C_{1-6}$ alkyl, $C_{3-6}$ cycloalkyl, $C_{3-6}$ heterocyclyl, $C_6$ aryl and $C_{5-6}$ heteroaryl; and said $R_2$ is substituted optionally with halogen.

**22.** The compound of any one of claims 1-21, or a stereoisomer, geometric isomer or tautomer, or a pharmaceutically acceptable salt, solvate, chelate, noncovalent complex or prodrug thereof, wherein $R_2$ is selected from $-CH_3$, $-CH_2CH_3$, $-CH(CH_3)_2$, $-CH_2CF_3$, cyclopropyl , cyclobutyl, phenyl and pyridyl; wherein $-CH_3$, $-CH_2CH_3$, $-CH(CH_3)_2$, $-CH_2CF_3$, cyclopropyl , cyclobutyl, phenyl and pyridyl are each optionally substituted with halogen.

**23.** The compound of any one of claims 1-22, or a stereoisomer, geometric isomer or tautomer, or a pharmaceutically acceptable salt, solvate, chelate, noncovalent complex or prodrug thereof, wherein $R_2$ is selected from $-CH_3$, $-CH(CH_3)_2$, cyclopropyl, phenyl and halogen substituted phenyl.

**24.** The compound of claim 1, or a stereoisomer, geometric isomer or tautomer, or a pharmaceutically acceptable salt, solvate, chelate, noncovalent complex or prodrug thereof, wherein the compound is selected from:

1) (2S)-1-(2-(5-isopropyl-3-methyl-2,4-dioxoimidazolidin-1-yl)-5,6-dihydrobenzo[f]imidazo[1,2-d] [1,4]oxazepin-9-yl)pyrrolidine-2-carboxamide;
2) (2S)-1-(2-(3-cyclopropyl -5-isopropyl-2,4-dioxoimidazolidin-1-yl)-5,6-dihydrobenzo[f]imidazo[1,2-d] [1,4]oxazepin-9-yl)pyrrolidine-2-carboxamide;
3) (2S)-1-(2-(5-isopropyl-2,4-dioxo-3-(2,2,2-trifluoroethyl)imidazolidine-1-yl)-5,6-dihydrobenzo[f] imidazo [1, 2-d] [1,4]oxazepin-9-yl)pyrrolidine-2-carboxamide;
4) (2S)-1-(2-(3-(4-fluorophenyl)-5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
5) (2S)-1-(2-(5-cyclopropyl -3-methyl-2,4-dioxoimidazolidin-1-yl)-5,6-dihydrobenzo[f]imidazo[1,2-d] [1,4] oxazepin-9-yl)pyrrolidine-2-carboxamide;
6) (2S)-1-(2-(3-cyclobutyl-5-isopropyl-2,4-dioxoimidazolidin-1-yl)-5,6-dihydrobenzo[f]imidazo[1,2-d] [1,4]oxazepin-9-yl)pyrrolidine-2-carboxamide;
7) (2S)-1-(2-(3-ethyl-5-isopropyl-2,4-dioxoimidazolidin-1-yl)-5,6-dihydrobenzo[f]imidazo[1,2-d] [1,4]oxazepin-9-yl)pyrrolidine-2-carboxamide;
8) (2S)-1-(2 -(5-difluoromethyl-3-methyl-2,4-dioxoimidazolidin-1-yl)-5,6-dihydrobenzo[f]imidazo[1,2-d] [1,4] oxazepin-9-yl)pyrrolidine-2-carboxamide;
9) (2S)-1-(2-(5-isopropyl-2,4-dioxo-3-phenylimidazolidine-1-yl)-5,6-dihydrobenzo[f]imidazo[1,2-d] [1,4] oxazepin-9-yl)pyrrolidine-2-carboxamide;
10) (S)-1-(2-((S)-5-isopropyl-2,4-dioxo-3-phenylimidazolidine-1-yl)-5,6-dihydrobenzo[f]imidazo[1,2-d] [1,4]oxazepin-9-yl)pyrrolidine-2-carboxamide;
11) (S)-1-(2-((R)-3-isopropyl-2,4-dioxo-3-phenylimidazolidine-1-yl)-3,6-dihydrobenzo[f]imidazo[1,2-d] [1,4]oxazepin-9-yl)pyrrolidine-2-carboxamide;
12) (2S)-1-(2-(5-isopropyl-2,4-dioxo-3-(4-(trifluoromethyl)phenyl)imidazolidine-1-yl)-5,6-dihydrobenzo[f]imidazo[1,2 -d] [1,4] oxazepin-9-yl)pyrrolidine-2-carboxamide;
13) (2S)-1-(2-(5-isopropyl-2,4-dioxo-3-propylimidazolidine-1-yl)-5,6-dihydrobenzo[f]imidazo[1,2-d] [1,4] oxazepin-9-yl)pyrrolidine-2-carboxamide;
14) (2S)-1-(2-(3-cyclopropyl -5-isopropyl)-2,4-dioxoimidazolidin-1-yl)-5,6-dihydrobenzo[f]imidazo[1,2-d] [1,4] oxazepin-9-yl)-2-methylpyrrolidine-2-carboxamide;
15) (2S)-1-(2-(3-cyclopropyl -5-isopropyl)-2,4-dioxoimidazolidin-1-yl)-5,6-dihydrobenzo[f]imidazo[1,2-d] [1,4] thiazepin-9-yl)-4,4-difluoropyrrolidine-2-carboxamide;
16) (2S)-1-(2-(5-isopropyl-3-(1-methyl-1H-1,2,4-triazol-5-yl)-2,4-dioxoimidazolidin-1-yl)-5,6 -dihydrobenzo[f] imidazo[1,2-d] [1,4]oxazepin-9-yl)pyrrolidine-2-carboxamide;
17) (2S,4S)-4-amino-1-(2-(3-cyclopropyl -5-isopropyl-2,4-dioxoimidazolidin-1-yl)-5,6-dihydrobenzo[f] imidazo [1,2-d] [1,4]oxazepin-9-yl)pyrrolidine-2-carboxamide;
18) (2S)-1-(2-(3-(2-fluorophenyl)-5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
19) (2S)-1-(2-(3-(4-chlorophenyl)-5-isopropyl-2,4-dioxoimidazolidin-1-yl)-5,6-dihydrobenzo[f]imidazo[1,2-d] [ 1,4] oxazepin-9-yl)pyrrolidine-2-carboxamide;
20) (2S)-4-(tertbutoxy)-1-(2-(3-cyclopropyl -5-isopropyl-2,4-dioxoimidazolidin-1-yl)-5,6-dihydrobenzo[f] imidazo [1,2] -d] [1,4]oxazepin-9-yl)pyrrolidine-2-carboxamide;
21) (2S)-1-(2-(3-cyclopropyl -5-isopropyl)-2,4-dioxoimidazolidin-1-yl)-5,6-dihydrobenzo[f]imidazo[1,2-d] [1,4] oxazepin-9-yl)-4,4-dimethylpyrrolidine-2-carboxamide;
22) (2S)-1-(2-(3-(2,2-difluoroethyl)-5-isopropyl-2,4-dioxoimidazolidin-1-yl)-5,6-dihydrobenzo[f]imidazo[1,2-d ] [1,4]oxazepin-9-yl)pyrrolidine-2-carboxamide;
23) (2S)-1-(2-(3,5-diisopropyl-2,4-dioxoimidazolidin-1-yl)-5,6-dihydrobenzo[f]imidazo[1,2-d] [1,4] oxazepin- 9-yl)pyrrolidine-2-carboxamide;
24) (2S)-1-(2-(3-cyclopropyl -5-isopropyl)-2,4-dioxoimidazolidin-1-yl)-5,6-dihydrobenzo[f]imidazo[1,2-d] [1,4] oxazepin-9-yl)-4-phenylpyrrolidine-2-carboxamide;
25) (2S)-1-(2-(3-(3-chloro5-cyanophenyl)-5-isopropyl-2,4-dioxoimidazolidin-1-yl)-5,6-dihydrobenzo[f]imidazo[1,2-d] [1,4] thiazepin-9-yl)pyrrolidine-2-carboxamide;
26) (2S)-1-(2-(3,5-diisopropyl-2,4-dioxoimidazolidin-1-yl)-5,6-dihydrobenzo[f]imidazo[1,2-d] [1,4]thiazepin- 9-yl)pyrrolidine-2-carboxamide;
27) (2S)-1-(2- (3-azetidin-3-yl)-5-isopropyl-2,4-dioxoimidazolidin-1-yl)-5,6-dihydrobenzo[f]imidazo[1,2-d ] [1,4] oxazepin-9-yl)pyrrolidine-2-carboxamide;
28) (2S)-1-(2-(3-methyl-2,4-dioxoimidazolidin-1-yl)-5,6-dihydrobenzo[f]imidazo[1,2-d] [1,4] oxazepin-9-yl)pyrrolidine-2-carboxamide;
29) (2S)-1-(2- (5-tertbutyl-3-methyl-2,4-dioxoimidazolidin-1-yl)-5,6-dihydrobenzo[f]imidazo[1,2-d] [ 1,4] oxazepin-9-yl)pyrrolidine-2-carboxamide;
30) (S)-1-(2-((R)-3-(4-fluorophenyl)-5-isopropyl-2,4-dioxoimidazolidin-1-yl)-5,6-dihydrobenzo[f]imidazo[1,2]-d] [1,4]oxazepin-9-yl)pyrrolidine-2-carboxamide;
31) (S)-1-(2-((S)-3-(4-fluorophenyl)-5-isopropyl-2,4-dioxoimidazolidin-1-yl)-5,6-dihydrobenzo[f]imidazo[1,2] -d] [1,4]oxazepin-9-yl)pyrrolidine-2-carboxamide;
32) (2S)-1-(2-((5R)-3-cyclopropyl -5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
33) (2S)-1-(2-((5S)-3-cyclopropyl -5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
34) (2S)-1-(2-(3-(2-hydroxyethyl)-5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
35) (2S)-1-(2-(3-(2-fluoroethyl)-5-isopropyl-2,4-dioxoimidazolidin-1-yl)-5,6-dihydrobenzo[f]imidazo[1,2-d] [1,4] oxazepin-9-yl)pyrrolidine-2-carboxamide;
36) (2S)-1-(2-(3-(2-(dimethylamino)ethyl)-5-)isopropyl-2,4-dioxoimidazolidin-1-yl)-5,6-dihydrobenzo[f]imidazo[1,2- d] [1,4]oxazepin-9-yl)pyrrolidine-2-carboxamide;
37) (2S)-1-(2-(3-cyclopropyl -5-isopropyl)-2,4-dioxoimidazolidin-1-yl)-5,6-dihydrobenzo[f]imidazo[1,2-d] [1,4] thiazepin-9-yl)pyrrolidine-2-carboxamide;
38) (2S)-1-(2-(3-(6-fluoropyridin-3-yl)-5-isopropyl-2,4-dioxoimidazolidin-1-yl)-5,6-dihydrobenzo[f]imidazo[1,2 -d] [1,4]oxazepin-9-yl)pyrrolidine-2-carboxamide;
39) (2S)-1-(2-(3-(5-fluoropyridin-3-yl)-5-isopropyl-2,4-dioxoimidazolidin1-yl)-5,6-dihydrobenzo[f]imidazo[1,2] -d] [1,4]oxazepin-9-yl)pyrrolidine-2-carboxamide;
40) (2S)-1-(2-(3-(3-cyano-5-fluorophenyl)-5-isopropyl-2,4-dioxoimidazolidin-1-yl)-5,6-dihydrobenzo[f]imidazo[1,2 -d] [1,4]oxazepin-9-yl)-4-fluorinepyrrolidine-2-carboxamide;
41) (2S)-1-(2-(3-(3-chloropyrimidin-2-yl)-5-isopropyl-2,4-dioxoimidazolidin-1-yl)-5,6-dihydrobenzo[f]imidazo[1,2 -d] [1,4]oxazepin-9-yl)pyrrolidine-2-carboxamide;
42) (2S)-1-(2-(3-cyclopentyl-5-isopropyl-2,4-dioxoimidazolidin-1-yl)-5,6-dihydrobenzo[f]imidazo[1,2-d] [1,4] oxazepin-9-yl)pyrrolidine-2-carboxamide;
43) (2S)-1-(2-(3-(cyanomethyl)-5-isopropyl-2,4-dioxoimidazolidin-1-yl)-5,6-dihydrobenzo[f]imidazo[1,2-d] [1,4] oxazepin-9-yl)pyrrolidine-2-carboxamide;
44) (2S)-1-(2-(5-isopropyl-3-methyl-2,4-dioxoimidazolidin-1-yl)-5,6-dihydrobenzo[f]imidazo[1,2-d] [1,4] thiazepin-9-yl)pyrrolidine-2-carboxamide;
45) (2S)-1-(2-(3-(2,2-difluoroethyl)-5-isopropyl-2,4-dioxoimidazolidin-1-yl)-5,6-dihydrobenzo[f]imidazo[1,2-d ] [1,4]thiazepin-9-yl)pyrrolidine-2-carboxamide;
46) (2S)-1-(2-(3-cyclopropyl -5-isopropyl-2,4-dioxoimidazolidin-1-yl)-3-vinyl-5,6-dihydrobenzo[f]imidazo[1,2-d] [ 1,4]oxazepin-9-yl)pyrrolidine-2-carboxamide;
47) (2S)-1-(3-chloro-2-(3-cyclopropyl -5-isopropyl-2,4-dioxoimidazolidin-1-yl)-5,6-dihydrobenzo[f]imidazo[1,2-d] [ 1,4]oxazepin-9-yl)pyrrolidine-2-carboxamide;
48) (2S)-1-(2-(3-cyclopropyl -5-(difluoromethyl)-2,4-dioxoimidazolidin-1-yl)-5,6-dihydrobenzo[f]imidazo[1,2-d] [1, 4]thiazepin-9-yl)pyrrolidine-2-carboxamide;
49) (2S,3S)-1-(2-(3-cyclopropyl -5-isopropyl-2,4-dioxoimidazolidin-1-yl)-5,6-dihydrobenzo[f]imidazo[1,2-d] [1,4] oxazepin-9-yl)-3- hydroxypyrrolidine -2-carboxamide;
50) (2S)-1-(2-(3-cyclopropyl -5-isopropyl-2,4-dioxoimidazolidin-1-yl)-3-methyl-5,6-dihydrobenzo[f]imidazo[1,2-d] [ 1,4]oxazepin-9-yl)-4-oxopyrrolidine-2-carboxamide;
51) (2S)-4-cyclohexyl-1-(2-(3-cyclopropyl -5-isopropyl-2,4-dioxoimidazolidin-1-yl)-5,6-dihydrobenzo[f]imidazo[1,2-d] [1,4]oxazepin-9-yl)pyrrolidine-2-carboxamide; and
52) (2S)-1-(2-(3-cyclopropyl -5-isopropyl-2, 4-dioxoimidazolidin-1-yl)-5, 6-dihydrobenzo[f] imidazo [1, 2-d] [1, 4] thiazepin-9-yl)-5-oxopyrrolidine-2-carboxamide.

**25.** A pharmaceutical composition, which comprises a therapeutically effective amount of at least one compound of any one of claims 1-24 or a stereoisomer, geometric isomer or tautomer, or a pharmaceutically acceptable salt, solvate, chelate, noncovalent complex or prodrug thereof and at least one pharmaceutical acceptable excipient.

**26.** The pharmaceutical composition of claim 25, wherein the weight ratio of the said compound or a stereoisomer, geometric isomer or tautomer, or a pharmaceutically acceptable salt, solvate, chelate, noncovalent complex or prodrug thereof and the pharmaceutical acceptable excipient is 0.0001-10.

**27.** The use of the compound of any one of claims 1-24 or a stereoisomer, geometric isomer or tautomer, or a pharmaceutically acceptable salt, solvate, chelate, noncovalent complex or prodrug thereof or the pharmaceutical composition of claims 25 or 26 for the preparation of a medicament.

**28.** The use of claim 27, wherein the said medicament is used to treat, prevent, delay or stop the occurrence or progression of cancer or cancer metastasis.

**29.** The use of claim 28, wherein the medicament is a PI3K inhibitor.

**30.** The use of claim 27, wherein the medicament is used for the treatment of the disease mediated by PI3K.

**31.** The use of claim 29 or 30, wherein the PI3K is PI3K$\alpha$, PI3K$\beta$, PI3K$\delta$ and /or PI3K$\gamma$.

**32.** The use of claim 31, wherein the PI3K is PI3K$\alpha$.

**33.** The use of claim 30, wherein the disease mediated by PI3K is cancer.

**34.** The use of claim 33, wherein the cancer is selected fromsarcoma, prostate cancer, breast cancer, pancreatic cancer, gastrointestinal cancer, colorectal cancer, thyroid cancer, liver cancer, adrenal cancer, glioma, endometrial cancer, melanoma, kidney cancer, bladder cancer, uterine cancer, vagina cancer, ovarian cancer, multiple myeloma, esophageal cancer, leukemia, brain cancer, oral and pharyngeal cancer, laryngeal cancer, lymphoma, basal cell carcinoma, polycythemia vera, essential thrombocythemia.

**35.** A method for the treatment or prevention of a disease mediated by PI3K, wherein the method comprising administering to said subject in need thereof a therapeutically effective amount of the compound of any one of claims 1-24 or the pharmaceutical composition of claim 25 or 26.

**36.** The method of claim 35, wherein the PI3K include PI3Kα, PI3Kβ, PI3Kδ and /or PI3Kγ.

**37.** The method of claim 35, wherein PI3K is PI3Kα.

**38.** The method of any one of claims 35 -37, wherein the disease mediated by PI3K is cancer.

**39.** The method of claim 38, wherein the cancer is sarcoma, prostate cancer, breast cancer, pancreatic cancer, gastrointestinal cancer, colorectal cancer, thyroid cancer, liver cancer, adrenal cancer, glioma, endometrial cancer, melanoma, kidney cancer, bladder cancer, uterine cancer, vagina cancer, ovarian cancer, multiple myeloma, esophageal cancer, leukemia, brain cancer, oral and pharyngeal cancer, laryngeal cancer, lymphoma, basal cell carcinoma, polycythemia vera, essential thrombocythemia.

**40.** A method for the treatment of cancer, comprising administering to subject in need thereof a therapeutically effective amount of the compound of any one of claims 1-24 or the pharmaceutical composition of claim 25 or 26, wherein the cancer is sarcoma, prostate cancer, breast cancer, pancreatic cancer, gastrointestinal cancer, colorectal cancer, thyroid cancer, liver cancer, adrenal cancer, glioma, endometrial cancer, melanoma, kidney cancer, bladder cancer, uterine cancer, vagina cancer, ovarian cancer, multiple myeloma, esophageal cancer, leukemia, brain cancer, oral and pharyngeal cancer, laryngeal cancer, lymphoma, basal cell carcinoma, polycythemia vera, essential thrombocythemia.

**41.** The method of claim 40, wherein the subject is human.

## INTERNATIONAL SEARCH REPORT

International application No.
**PCT/CN2020/126359**

**A. CLASSIFICATION OF SUBJECT MATTER**

C07D 498/04(2006.01)i; C07D 498/14(2006.01)i; C07D 519/00(2006.01)i; A61P 35/00(2006.01)i; A61K 31/4188(2006.01)i; A61K 31/4196(2006.01)i; A61K 31/4162(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNPAT, WPI, EPODOC, CNKI, STN(CAPLUS), C07D498, 贝达药业, 咪唑烷酮, 氧氮杂卓, PI3K, 肿瘤, 癌症, tumor, cancer, benzoxepin.

**C. DOCUMENTS CONSIDERED TO BE RELEVANT**

| Category* | Citation of document, with indication, where appropriate, of the relevant passages | Relevant to claim No. |
|---|---|---|
| X | CN 102762576 A (F. HOFFMANN-LA ROCHE AG) 31 October 2012 (2012-10-31) claims 8 and 9, description paragraphs [0026]-[0033], [0107] and [0108], pages 77, 78, 94 and 108 | 1-41 |
| X | CN 103562210 A (F. HOFFMANN-LA ROCHE LTD.) 05 February 2014 (2014-02-05) claims 19-27, description page 80 | 1-41 |
| A | CN 107995911 A (HOFFMANN-LA ROCHE INC.) 04 May 2018 (2018-05-04) claims 7-11, description page 16 | 1-41 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 December 2020** | **29 January 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/126359**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **35-41**
because they relate to subject matter not required to be searched by this Authority, namely:

[1] The subject matter of claims 35-39 is a method for treatment and/or prophylaxis for a patient having a PI3K-mediated disease, and the subject matter of claims 40 and 41 is a method for treating cancer, thus relating to the living human or animal body, and belonging to treatment methods for diseases. The present report is provided on the basis of a use in drug preparation.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/126359**

| Patent document cited in search report | Publication date (day/month/year) | Patent family member(s) | Publication date (day/month/year) |
|---|---|---|---|
| CN 102762576 A | 31 October 2012 | IL 239081 D0 | 30 July 2015 |
| | | CA 2772691 A1 | 31 March 2011 |
| | | KR 20120065431 A | 20 June 2012 |
| | | AR 078187 A1 | 19 October 2011 |
| | | TW I423980 B | 21 January 2014 |
| | | US 8785626 B2 | 22 July 2014 |
| | | HK 1174629 A1 | 18 March 2016 |
| | | EP 2711368 A1 | 26 March 2014 |
| | | MX 2012003591 A | 19 April 2012 |
| | | ES 2570569 T3 | 19 May 2016 |
| | | WO 2011036280 A1 | 31 March 2011 |
| | | CO 6491026 A2 | 31 July 2012 |
| | | AU 2010299816 C1 | 01 February 2018 |
| | | PE 20121025 A1 | 06 August 2012 |
| | | DK 2483278 T3 | 13 January 2014 |
| | | RS 53164 B | 30 June 2014 |
| | | EP 2483278 B1 | 25 December 2013 |
| | | MA 33531 B1 | 01 August 2012 |
| | | US 9546178 B2 | 17 January 2017 |
| | | IL 217558 D0 | 29 February 2012 |
| | | US 2017081341 A1 | 23 March 2017 |
| | | US 8343955 B2 | 01 January 2013 |
| | | US 8242104 B2 | 14 August 2012 |
| | | IL 239081 A | 31 May 2018 |
| | | CN 102762576 B | 22 April 2015 |
| | | US 9670228 B2 | 06 June 2017 |
| | | US 2011076292 A1 | 31 March 2011 |
| | | NZ 597833 A | 31 January 2014 |
| | | EP 2845592 A1 | 11 March 2015 |
| | | HK 1211929 A1 | 03 June 2016 |
| | | AU 2010299816 A1 | 02 February 2012 |
| | | JP 5625101 B2 | 12 November 2014 |
| | | US 9198918 B2 | 01 December 2015 |
| | | CA 2772691 C | 03 October 2017 |
| | | US 2013079331 A1 | 28 March 2013 |
| | | PL 2483278 T3 | 30 May 2014 |
| | | RU 2600927 C2 | 27 October 2016 |
| | | RU 2654068 C1 | 16 May 2018 |
| | | CL 2012000754 A1 | 07 September 2012 |
| | | JP 2013505917 A | 21 February 2013 |
| | | EC SP12011755 A | 30 May 2012 |
| | | SI EP2483278 T1 | 31 March 2014 |
| | | US 2014058098 A1 | 27 February 2014 |
| | | US 2012244149 A1 | 27 September 2012 |
| | | KR 101428346 B1 | 07 August 2014 |
| | | US 2016052933 A1 | 25 February 2016 |
| | | EP 2483278 A1 | 08 August 2012 |
| | | JP 5540101 B2 | 02 July 2014 |
| | | EP 2711368 B1 | 23 March 2016 |
| CN 103562210 A | 05 February 2014 | BR 112013024122 A2 | 24 September 2019 |

Form PCT/ISA/210 (patent family annex) (January 2015)

**INTERNATIONAL SEARCH REPORT**
**Information on patent family members**

International application No.
**PCT/CN2020/126359**

| Patent document cited in search report | Publication date (day/month/year) | Patent family member(s) | Publication date (day/month/year) |
|---|---|---|---|
| | | EP 2688891 B1 | 15 November 2017 |
| | | US 9090628 B2 | 28 July 2015 |
| | | CA 2825966 A1 | 27 September 2012 |
| | | JP 6001635 B2 | 05 October 2016 |
| | | WO 2012126901 A1 | 27 September 2012 |
| | | HK 1194383 A1 | 24 February 2017 |
| | | MX 340013 B | 22 June 2016 |
| | | US 2012245144 A1 | 27 September 2012 |
| | | EP 2688891 A1 | 29 January 2014 |
| | | KR 20140032383 A | 14 March 2014 |
| | | MX 2013009934 A | 01 October 2013 |
| | | CN 103562210 B | 25 May 2016 |
| | | RU 2013143747 A | 27 April 2015 |
| | | JP 2014509611 A | 21 April 2014 |
| CN 107995911 A | 04 May 2018 | US 2017002022 A1 | 05 January 2017 |
| | | CN 107995911 B | 04 August 2020 |
| | | EP 3317283 A1 | 09 May 2018 |
| | | JP 6522807 B2 | 29 May 2019 |
| | | HK 1254517 A1 | 19 July 2019 |
| | | JP 2018519304 A | 19 July 2018 |
| | | WO 2017001658 A1 | 05 January 2017 |
| | | US 9643980 B2 | 09 May 2017 |
| | | EP 3317283 B1 | 03 April 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017001658 A


### Non-patent literature cited in the description


- **MA K ; CHEUNG SM ; MARSHALL AJ.** *Cell Signal,* 2008, vol. 20, 684-694
- **VANHAESEBROECK B ; WATERFIELD MD.** *Exp Cell Res,* 1999, vol. 253, 239-254
- **WU P ; LIU T ; HU Y.** *Curr Med Chem,* 2009, vol. 16, 916-930
- **FALASCA M ; T. MUFFUCCI.** *Biochem Soc Trans,* 2007, vol. 35, 211-214
- **SCHU P ; TAKEGAWA. K ; FRY. M.** *Science,* 1993, vol. 260, 88-91
- **KONG D ; YAMORI T.** *cancer Sci,* 2008, vol. 99, 1734-1740
- **STEELMAN. LS ; CHAPPELL. WH ; ABRAMS. SLET.** *Aging,* 2011, vol. 3, 192-222
- **DEJAN JURIC.** *cancer Res,* 2012, vol. 72, 1
- Design of Prodrugs. Elsevier, 1985
- **KOLA I ; LANDIS J.** *Nat Rev Drug Discov.,* 2004, vol. 3 (8), 711-5